# EUROPEAN PATENT SPECIFICATION

(11) **EP 0 934 530 B1**
(45) Date of publication and mention of the grant of the patent: **27.06.2007**
(21) Application number: 97939899.7
(22) Date of filing: 05.09.1997
(51) Int. Cl.: G01N 33/574, G01N 33/577, C07K 16/30

(54) **IMMUNOLOGICAL COMPOSITION FOR DETECTION OF BLADDER CANCER AND METHOD OF USING SAME**
IMMUNOLOGISCHE ZUSAMMENSETZUNG ZUM NACHWEIS VON BLASENKREBS UND VERFAHREN ZU IHRER VERWENDUNG
COMPOSITION IMMUNOLOGIQUE DE DETECTION DU CANCER DE LA VESSIE, ET SON MODE D'UTILISATION

(30) Priority: 19.09.1996 US 26361
(43) Date of publication of application: 11.08.1999
(73) Proprietor: Diagnocure Inc., Ste-Foy, Quebec G1V 2K8 (CA)
(72) Inventor: FRADET, Yves, Syllery, Quebec G1S 3S1 (CA); LOCKHART, Campbell, Quebec, Quebec G1S 4V5 (CA); PARENT, Carmen, Quebec, Quebec G2K 1N6 (CA); PROULX, Sonia, Val Bélair, Quebec G3K 1P0 (CA)
(74) Representative: Bergstrand, Mikael Gudmundsson
(86) International application number: PCT/CA1997/000645
(87) International publication number: WO 1998/012564

(56) References cited:
- US-A- 5 039 611
- P.M. SAGERMAN ET AL.: "Enhanced detection of bladder cancer in urine cytology with lewis X, M344 and 19A211 antigens" ACTA CYTOLOGICA, vol. 38, no. 4, 1 July 1994, MINEOLA NY USA, pages 517-523, XP002050163 cited in the application
- G. GAMBUS ET AL.: "Detection of the MUC2 apomucin tandem repeat with a mouse monoclonal antibody" GASTROENTEROLOGY, vol. 104, no. 1, 1 January 1993, ORLANDO FL USA, pages 93-102, XP002050164 cited in the application

## Description

### FIELD OF THE INVENTION

The present invention relates in general to cancers and more particularly to bladder cancer. More specifically, the present invention relates to non-invasive immunocytological methods to diagnose, assess or prognose a human patient afflicted with bladder cancer, kits containing immunological compositions for the non-invasive specific and sensitive detection of bladder cancer; and methods of preparing cells from a urine sample for immunocytological analysis.

### BACKGROUND OF THE INVENTION

Each week in 1997, an average of 26 Canadians will die of bladder cancer, a total of 1350 lives lost (National Cancer Institute of Canada. Canadian Cancer Statistics 1997. Ottawa: National Cancer Institute of Canada, 1997). This year, an estimated 4500 new cases will be diagnosed, 3400 in men and 1100 in women, representing almost five percent of all new cancers diagnosed (National Cancer Institute of Canada. Canadian Cancer Statistics 1997. Ottawa: National Cancer Institute of Canada, 1997). The ratio of deaths to cases, 0.30, is higher than for either cancer of the prostate or breast cancer (National Cancer Institute of Canada. Canadian Cancer Statistics 1997. Ottawa: National Cancer Institute of Canada, 1997).

Of all cancers found in the bladder, 99 percent are carcinomas, and more than 90 percent are transitional cell carcinomas. One of the unique clinical features of this cancer is the high proportion, more than 80 percent, that are superficial, non-invasive tumors at the time of presentation (Huben RP, 1996, Sem Urol Oncol., 14:23-9). Another notable feature of transitional cell carcinoma is the high recurrence rate after initial therapy, at least 70 percent (Huben RP, 1996, Sem Urol Oncol, 14:23-9.; Allard P et al., 1995, Clin Cancer Res, 1:1195-1202).

This combination of low stage and grade at presentation, along with a very high recurrence rate, means that patients with transitional cell carcinoma of the bladder will survive for a long time, and require regular follow-up to detect and deal with recurrences. Currently, two tests are available for the initial diagnosis, and subsequent follow-up, of patients with bladder cancer, urinary cytology and cytoscopy with biopsy.

From the patient point of view, urinary cytology is a simple painless test. Moreover, with standardization of specimen collection, rapid processing, and other improvements in laboratory techniques, urinary cytology has become highly specific, with a false-positive rate usually reported as zero (Schwalb DM et al., 1993, J. Urol.150:1751-6). The rare patient who has positive cytology without cystoscopic abnormality will almost invariably display a cancer, given sufficient investigation or follow-up time (Schwalb DM et al., 1993, J. Urol.150:1751-6). However, the sensitivity of cytology is poor, typically about 30%.

Briefly, urinary cytology consists in the analysis of the structural appearance of cells obtained from urine. The cells are stained with a standard reagent and analysed under a microscope. Numerous criteria are used to identify malignant cancer cells and, for a cell to be considered as such, it must display a combination of several of these criteria, some of which may be highly dependent on the particular pathologist's judgment. The reliability of a diagnosis based on cytology also suffers because of additional factors, including the slow turnover of urothelial cells that may result in few cells being available for analysis in any given sample (Murphy WM., 1990, 21:886-96). As well, the cytological architecture of the common low-grade papillary tumors is not easily distinguishable from that of normal cells. In terms of monitoring mechanism, routine urinary cytologies suffer from a lack of precision, with sensitivity varying according to the phase of histological grade of the tumor.

The other test available for diagnosis and monitoring is cystoscopy with biopsy (either of visible lesions, or at random sites if no lesion is visible) (Schwalb DM et al., 1993, J. Urol.150:1751-6). Although this procedure is highly accurate, it is considered the Gold standard in bladder cancer diagnostics, it does involve substantial discomfort, requiring some form of anesthesia. Cystoscopy is also expensive. Of importance, it is estimated that more than 50% of patients who suffer from the superficial mode will eventually go on the develop more serious disease (invasive mode). Cystoscopy is thus the standard method used in diagnosing this type of cancer and is highly accurate. However, it is an invasive, painful procedure for patients. In addition, it is costly, and its use is limited to diagnosing tumors which permit visualization.

There thus remains a need for early identification of bladder cancer using a non-invasive method. Early detection of bladder cancer could translate in an increase in the curing rate. There also remains a need for tools and non-invasive methods for diagnosis of bladder cancer which display a sensitivity between 95% and 100%.

Clinical and fundamental research have shown that bladder tumors present themselves in two (2) ways: superficial tumors, known as papillary tumors Ta/T1 and invasive tumors, known as tumors T2/T3, depending on the degree of invasion.

More than 75% of patients present the superficial papillary mode, a transitional variety which is located on the surface mucus membrane of the bladder. The risk of recurrence is higher than 50%. Patients who have received treatment for this type of cancer are regularly monitored using the two tests mentioned above, cytology and routine cystoscopy (4 - 5 times per year during the first 2 years, 2 times per year thereafter). As discussed previously, urinary cytology, the only existing non-invasive test, is only slightly sensitive, capable of detecting less than 30% of superficial tumors.

In view of the pain level associated with routine cytoscopy and the lack of sensitivity associated with cytology, there remains a need for a biological marker which would provide sensitivity and specificity to a non-inVasive method for bladder cancer-diagnostic.

The M344 and 19A211 antigens have been described in USP 5, 039,611 and in Allard et al., 1995 (Clin. Cancer. Res., 1:1195-1202). The M344 antigen is present on a cancer mucin highly restricted to early forms of bladder cancer and also to pre-malignant cells responsible for tumor recurrence. M344 monoclonal antibody (mAb) has also been shown to react with a mucin antigen of the urinary bladder (MAUB; Bergeron et al., 1996, J. Biol. Chem. 271:6933-40). M344 mAb apparently reacts with the same antigen as LDQ10, albeit with a higher degree of glycosylation. The 19A211 antigen is also expressed in superficial tumors and early forms of invasive bladder cancer and is part of the family of carcinoembryonic antigens (CEA). Other markers associated with bladder cancer have also been identified. (Lewis X is one example) (Sagerman et al., 1994, Acta Cytology 38:517-523; Golijanin et al., 1995, Urology 46:173-177).

Monoclonal antibody LDQ10 , an IgM, reacts with deglycosylated colon cancer mucin and with a synthetic peptide encompassing the MUC2 tandem repeat sequence. LDQ10 has also been shown to react with colorectal, stomach, pancreatic and breast cancers (Gambus et al., 1993, Gastroentererology 104:93-102). LDQ10 was shown to react strongly with T-MGH-U3, a superficial bladder cancer cell line grown as a tumor in nude mice (Bergeron et al., 1996).

However, the use of such markers in a simple, specific, sensitive and non-invasive method of bladder cancer diagnosis from a urine sample has yet to be described. The published and/or commercial tests based on the use of potential bladder tumor markers suffer especially from their lack of sensitivity and specificity in detecting low-grade tumors. Soloway et al., 1996, (J. of Urol. 156:363-7) describe a quantitative non-invasive immuno assay having an overall sensitivity of 70% (100% sensitivity for invasive bladder cancer). In a comparison of the Bard bladder tumor associated antigen (BTA) test to voided urine cytology (VUC) and bladder wash cytology (BW) in patients suspected of having bladder cancer, Legh et al., 1996 (J. of Urol. 155:492A, Abstract #726) report that the overall sensitivity for BTA and cytology (VUC and/or BW), was 64% and 28%, respectively. Once again, the tests are less sensitive for the lower grade tumors. The use of various tumor markers, such as CEA has also been evaluated (Van aken et al., USP 5,591,830). Therein, it is stated that CEA is usually restricted to advanced tumors and that urinary infections has been shown to cause false positives, raising doubts about the validity of an approach of bladder cancer diagnostic based on the detection of tumor markers (Van aken et al., USP 5,591,830).

Diagnosis and monitoring of bladder cancer would therefore benefit immensely from the introduction of a non-invasive test which utilizes markers offering a high degree of sensitivity and specificity, comparable if not superior, to current tests. There also remains a need for tools and tests which significantly increase the sensitivity towards low-grade tumors. In addition, there remains a need for early identification of bladder cancer using a non-invasive method that could decrease the mortality rate of patients affected by this disease. The present invention seeks to meet these and other needs.

The present description refers to a number of documents.

### SUMMARY OF THE INVENTION

This invention concerns the discovery of a non-invasive, investigative test which is not only more effective than cytoscopy or a biopsy for the detection of bladder cancer, but is also simpler to manipulate. In one preferred embodiment, the non-invasive test is carried out on shedded cells from the bladder. In yet another preferred embodiment, the test is carried out on a urine sample. In one embodiment, the non-invasive investigative test combines immunocytological and classical cytological analyses of cells obtained from a test sample to achieve a sensitivity which is superior to that of described non-invasive tests. Preferably, the sensitivity is higher than 80%, more preferably higher than 90% and even more preferably between 95% and 100%.

The invention also relates to an immunological composition comprising monoclonal antibodies (mAb) recognizing antigens associated with bladder cancer. In one preferred embodiment, the invention relates to a composition comprising mAbs which acts on different grades of tumors (i.e. pre-cancer, low-grade, intermediate grade or markers of invasive tumors).

Surprisingly, the combination of monoclonal antibodies M344 (having high specificity for a mucin-like marker selectively expressed on the non-invasive low-grade bladder tumors), and 19A211 (recognizing a CEA family member) and a further molecule recognizing a marker expressed in bladder cancer, was shown to be capable of significantly increasing the sensitivity of tumor detection in immunocytology. Further, the present invention provides a good complement to existent urinary tests to detect low-grade bladder tumors. The immunological test more specifically detects low-grade tumors while the classical cytology more specifically detects high grade or high stage tumors. In one preferred embodiment, the further molecule is a monoclonal antibody. In a particularly preferred embodiment, the monoclonal antibody is LDQ10.

In one particular embodiment, the composition of monoclonal antibodies are labeled with different fluorochrome that can permit a selective adjustment of the threshold of detection with the non-specific background reactivity of one antibody of the composition. Therefore, such an immunological combination provides a useful complement to conventional cytology because it enables the sensitive and specific detection of tumors of different stages and grades and more especially of low stages or grades. In addition, this combination provides a mean to estimate the risk of recurrence and tumor rate in bladder cancer. Thus, in a preferred embodiment the composition comprises mAbs M344, 19A211 and LDQ10. The hybridomas producing M344 and 19A211 have been deposited at the American Type Culture Collection (ATCC, 12301 Parklawn Drive, Rockville, Md) on March 31, 1988 and granted accession numbers ATCC HB9678 and ATCC HB9679, respectively. The hybridoma producing LDQ10 has been deposited at the ATCC on August 29, 1997 and granted ATCC accession number ATCC HB12417.

The present invention further relates to an immunological composition for the simple, sensitive and specific detection of bladder cancer which opens the way to a characterization of the type of bladder cancer involved, and possibly to a prognosis thereof.

The invention also relates to the combination of the compositions of the present invention and/or the methods of detection of bladder cancer of the present invention with other markers such as morphometry, proliferation rates and karyotyping.

In addition, the present invention relates to immunocytological methods for detection of bladder cancer on cells collected from a voided urine sample comprising the use of the immunocytological compositions of the present invention.

Further, the present invention pertains to methods for preparing cells shedded from the bladder for an immunocytological assay for the detection and/or prognosis and/or assessment of urogenital cancer. In one preferred embodiment, the cells are obtained from a voided urine sample and the method of preparing the cells for immunocytology enables a significant reduction in non-specific fluorescence and morphological artifacts which are frequent in urinary cytology.

The present invention therefore seeks to provide a diagnostic method which is more sensitive than conventional cytology and has the advantage of being a more effective alternative to invasive methods such as cystoscopy. The present invention further seeks to provide an immunocytological composition which enables a non-invasive method of diagnosing, assessing or even prognosing bladder cancer which is as effective as a cystoscopy, but is also simpler since dosage is performed on shedded cells from the bladder.

The present invention also seeks to provide a method for the estimation of the risk of recurrence and tumor rate in bladder cancer. In one embodiment, the method comprises the steps of: a) isolating shedded cells from the bladder of a patient; b) fixing said cells to preserve their morphological characteristics; c) reacting said cells with an antibody cocktail comprising mAbs M344, LDQ10, and 19A211; and d) estimating the risk of recurrence and the tumor rate based on the reactivity of said mAbs with said tumor. The risk of recurrence of the tumor can be determined by comparing the amount of reactivity of M344, which is associated with an increase in the tumor rate. The reactivity of 19A211 with the tumor is associated with an increase in first recurrence hazard and in recurrence rate.

The present invention also seeks to provide a diagnostic kit, comprising the above-mentioned immunocytological composition and methods.

The invention further seeks to provide a fixative solution and a biphasic method of fixing cells which protects and maintains the morphological features of the cells, increases the recovery thereof from the biological sample, and reduces and/or prevents the formation of crystals in the biological and test sample.

In addition, the present invention seeks to provide a method of preparation of cells enabling the performance of both an immunological assay and routine cytology on the same cell preparation. More particularly, the fixed cells blotted on microscope slides are prepared for immunofluorescence by submitting them to a modified Papanicolaou procedure, to yield cells decorated with one or more antibodies. Following immunofluorescence, the slides can be further treated so as to enable a classical cytological analysis of the previously decorated cells.

As previously mentioned, the immunological test samples of the present invention include shedded cells from the bladder. Such shedded cells can be obtained in biological samples such as by bladder washings, biopsies and in voided urine. The term urine sample, as used herein, is meant to cover a biological sample containing shedded cells from the bladder. It shall also be understood by the person of ordinary skill that the test sample should be interpreted as also including total cells extracts, membrane fractions, or purified fractions therefrom obtained from the shedded cells. It shall be clear that the particular type of test sample to be used in the herein-described methods will vary based on the assay format, particularities of the detection method, as well as the origin of the cells and specific type of test sample to be assayed.

The cells can thus, in accordance with one aspect of the present invention, be used directly to assess the presence of the cancer markers which are targeted. Alternatively, the cells can be further processed to obtain membrane fractions or purified fractions therefrom. Such fractions can be used in accordance with aspects of the present invention to assess the presence of the targeted cancer markers. Methods of preparing membrane fractions from purified cells are well known to the person of ordinary skill. Such methods should maintain the integrity of the cancer markers to which the antibodies are targeted.

Method of lysing the cells and preparing membranes therefrom are well known in the art and can be readily adapted to meet particular needs. Representative examples of a cells lysing methods and methods of preparing membranes can be found for example in Campbell, 1984 (Monoclonal Antibody Technology: Laboratory Techniques in Biochemistry and Molecular Biology, Elsevier Science Publisher, Amsterdam, The Netherlands) and in Harlow et al., 1988 (in: Antibody - A Laboratory Manual, CSH Laboratories). It should be understood that the immunological composition of the invention can be used on membrane fractions or further purified preparation thereof, provided that the cancer markers which are targeted by the compositions have maintained their property of interaction therewith. Such further purification of the membrane fractions can be carried out according to conventional protein/glycosylated protein purification methods, such as gel filtration, ion exchange chromatography and affinity purification.

It will also be understood that the biological sample should contain cells expressing the antigen targeted by the immunological composition used. In addition to bladder cancers, LDQ10 shows strong reactivity with a majority of colorectal, stomach, pancreatic and breast cancers. Similarly, M344 is also reactive with some prostatic, endometrial, colon and breast carcinomas and 19A211 with some colon and breast carcinomas (Carlos Cordon-Cardo et al., 1992, Am. J. Pathol., 140:375-85).

The cocktail of antibodies of the present invention could therefore be a component of a diagnostic test for some cancers other than bladder, and also a component of cancer therapeutic tools such as targeted chemotherapy or vaccines. Since the M344 and LDQ10 antibodies react with mucins, expressed by definition by cells associated with mucus, it follows that the biological sample should contain mucus and/or cells expressing a mucin recognized by an antibody used. Such biological samples therefore include without being limited thereto (urine, bladder washings, prostate biopsies, cervical secretions, bronchial aspirate or secretions, sputum and feces). Preferably, the biological sample will contain cells expressing cancer-associated markers selected from the group consisting of MUC2, MAUB and CEA.

It will be understood that the type of biological sample used will be dependent on the particular disease to be tested. For example, urine will be preferred for the detection of urogenital cancers or disorders affecting mucin or CEA glycosylation. Similarly, feces will be preferred for assessing colon cancer and colon disorders affecting mucin or CEA glycosylation. Since the antibody cocktail of the present invention also recognizes more cells in a benign prostate hyperplasia (BPH), it could possibly be used to diagnose this disease as well as prostate cancer.

The antibodies employed in the present invention include monoclonal as well as fragments of these antibodies. The invention further includes the use of single chain antibodies. Antibody fragments which contain the idiotype of the molecule can be generated by known techniques. For example, such fragments include but are not limited to: the F(ab')₂ fragment; the Fab' fragments, Fab fragments, and Fv fragments.

Of special interest to the present invention are M344, LDQ10, and 19A211 antibodies which are produced in humans, or are "humanized" (i.e. non-immunogenic in a human) by recombinant or other technology. Humanized antibodies can be produced, for example by replacing an immunogenic portion of an antibody with a corresponding, but non-immunogenic portion (i.e. chimeric antibodies) (Robinson, R.R. et al., International Patent Publication PCT/US86/02269 (WO87/02671); Akira, K. et al., European Patent Application 184,187; Taniguchi, M., European Patent Application 171,496. Reviews on humanized chimeric antibodies include Morrison, S.L., 1985, Science 229:1202-1207 and Oi, V.T. et al., 1986, BioTechniques 4:214.

In general, techniques for preparing monoclonal antibodies and hybridomas are well known in the art (Campbell, 1984, supra; Harlow et al., 1988, supra; and St. Groth et al., 1980, J. Immunol. Methods 35:1-21. In general, techniques for purifying monoclonal antibodies are also well known in the art (Campbell, 1984, supra; Harlow et al., 1988, supra). Non-limiting examples of monoclonal antibody purification methods include ammonium sulfate precipitation, ion exchange chromatography and HPLC. Monoclonal antibodies can also be produced by bioreactor such as the hollow fiber cell culture system described in (the Unisyn instruction manual). For example, using this hollow fiber culture technology, choosing a hollow fiber membrane having a molecular weight cut off of 35.000, 1 x 10⁸ cells of hybridoma was introduced into the bioreactor. The hybridoma was grown in PFHM-11 media (GIBCO, BRL) with PEN/STREP (GIBCO/BRL).

In another embodiment of the present invention, the above-described antibodies are detectably labeled. Such labels can be radioisotopes, affinity labels (i.e. biotin, avidin), enzymatic labels (i.e. peroxidase, alkaline phosphatase) fluorescent labels (i.e. fluorescein, FITC, rhodamine, Texas Red^{®}). Methods of labelling antibodies are well known in the art (Campbel, 1984, supra; Harlow et al., 1988, supra). The labelled antibodies of the present invention can be used for *in vitro, in vivo,* and *in situ* assays to identify and/or target cells or tissues which express a specific cancer marker.

In another embodiment of the present invention the above-described antibodies are immobilized on a solid support. Examples of such solid supports are well known in the art and include plastics such as polycarbonate, complex carbohydrates such as agarose and sepharose, acrylic resins, polyacrylamide and latex beads. Techniques for coupling antibodies to such solid supports are well known in the art (Campbell, 1984, supra; Harlow et al., 1988, supra ; and Jacoby et al., 1974, Meth. Enzym. 34). The immobilized antibodies can be used according to well known methods in numerous types of assays including immunochromatography.

The conditions for incubating an antibody with a test sample is dependent on a number of parameters the assay format employed, the detection methods used, and intrinsic properties of the antibody used in the assay. It should be understood that the conditions are aimed at allowing a sufficient amount of immunocomplexes to form so as to be detectable. A person of ordinary skill will recognize that any one of the commonly available immunological assay formats (such as radioimmunoassays, enzyme-linked immunosorbent assays and the like) can be readily adapted to employ the antibodies of the present invention. Once again, reference to Campbell, 1984, and Harlow et al., 1988, supra, is made, as well as to USP 5,591,830 and references therein. It should also be clear that a number of the above-mentioned assays are amendable to quantification and even to automation.

The diagnostic and screening methods of the invention find relevance in the follow-up of patients having had surgical treatment to remove superficial bladder tumors, for a patient suspected of being at risk for developing based on family history, or on previous occurrence of the disease or simply for a patient desiring a diagnostic screening per se. In view of the high sensitivity, the screening method of the present invention, a presymptomatic diagnosis of bladder cancer is possible using the antibody cocktail mentioned above. Early diagnosis has the potential as to maximize the possibility of intervening in the early stage of the disease and significantly contributing to the prognosis thereof.

The fixative solution is an important component of the cell preparation method of the present invention, as it performs at least two functions. It preserves the morphological characteristics of the cells and the availability and functionality of the biomarkers of cells. In addition, it favors an efficient filtering in the sample-fixative mixture, enabling a good retention of the cells on the filter. One of the biological sample is a urine sample, in addition to cells, the sample also comprises protein and crystals. In an especially preferred embodiment, the fixative of the present invention which aims at protecting and maintaining the morphological characteristics of these cells, preferably also aims at reducing or at least preventing a detrimental formation of crystals. More preferably the fixative solution will inhibit the formation of crystals and solubilize crystals already present in the sample. It would be clear to a skilled artisan that the formation of crystals could have significant detrimental effects on the morphology of the cells and the optical analysis, following staining of the cells, etc. It shall be understood that the inhibition of crystals formation refers to the formation of crystals under the conditions which are described herein for urine sample preparation, slide preparation, and staining.

It shall be understood that not all crystals and forms may be inhibited or solubilized. The fixative aims at reducing the presence of crystals which comprise cations such as calcium and magnesium which can form during the preparation of the cells when different buffers and salts are added to the biological sample. The fixing of cells according to the present invention is biphasic, comprising a pre-fixing step in 50% ethanol and a fixing step per se, in the presence of buffer, salt and PEG.

Ethanol (50%) is used in the pre-fixing solution, serves as an inhibitor protein degradation, as a bactericidal substance and acts to dissolve some crystals present in the biological sample. The fixing solution in the second step contains a buffer, a salt in addition to PEG which contributes in maintaining morphological characteristics of the cells. Further, other buffers well know in the art are substitute for MOPS. Representative examples thereof include HEPES and PIPES (Sigma Chemical Co. Catalog). Although NaCl is used to increase the ionic strength of the buffer, other salts can be used, as well known to the person of ordinary skill.

It shall be understood that although it is preferable to carry the immunogical analysis and traditional cytological on the same slide, two different slides can also be used. It should be stressed that since the number of cells present on a slide is critical for the statistical significance of the test, the use of a single slide can be a critical factor, should a biological sample have too low a concentration of cells. It shall also be noted that cells potentially displaying a low-stage tumor Phenotype (Ta) and primarily recognized by M344 are especially scarce.

The filtration of cells which is improved by the components of the fixative solution is carried out preferably using polycarbonate filters having a pore size of approximately 8 micrometres (microns). Equivalent filters should be readily identified by a person of ordinary skill.

In order to obtain statistically relevant data using the method of bladder cancer diagnostic of the present invention which, in a preferred embodiment combines immunocytological and conventional cytological methods, a minimum of 500 cells should be present on the microscopic slides. Preferably, there should be between a 1000 and 5000 cells on the slide, more preferably more than 4000 cells.

The kit can comprise: i) a first container means containing an above-described antibody or antibody cocktail, and ii) second container means containing a conjugate comprising a binding partner of the antibody and a label.

In another preferred embodiment, the kit further comprises one or more other containers comprising one or more of the following: wash reagents and reagents capable of detecting the presence of bound antibodies. Examples of detection reagents include, but are not limited to, labeled secondary antibodies, or in the alternative, if the primary antibody is labeled, the chromophoric, enzymatic, or antibody binding reagents which are capable of reacting with the labeled antibody.

In detail, a compartmentalized kit includes any kit in which reagents are contained in separate containers. Such containers include small glass containers, plastic containers or strips of plastic or paper. Such containers allow the efficient transfer of reagents from one compartment to another compartment such that the samples and reagents are not cross-contaminated and the agents or solutions of each container can be added in a quantitative fashion from one compartment to another. Such containers will include a container which will accept the test sample, a container which contains antibody cocktail used in the assay, containers which contain wash reagents, and containers which contain the reagents used to detect, bound antibody, or the like.

In another preferred embodiment, the kit further comprises a container comprising fixing solution, buffer, cells staining solutions and the like.

One skilled in the art will readily recognize that the antibodies and method of detecting bladder cancer described in the present invention can readily be incorporated into one of the established kit formats which are well known in the art.

Suitable pharmaceutical carriers are described in the most recent edition of Remington's Pharmaceutical Sciences, Olsnes, S. et al., 1980, 16th Ed., a standard reference text in this field.

The antibodies employed in this invention may be advantageously utilized in combination with other monoclonal or murine and chimeric antibodies, fragments and regions, or with lectins, receptor ligands, or lymphokines or hemopoietic growth factors, etc., (termed hereinafter "molecules") which serve to increase the number or activity of effector cells which interact with the antibodies.

### BRIEF DESCRIPTION OF THE DRAWINGS

Having thus generally described the invention, reference will now be made to the accompanying drawing, showing by way of illustration a preferred embodiment thereof, and in which:

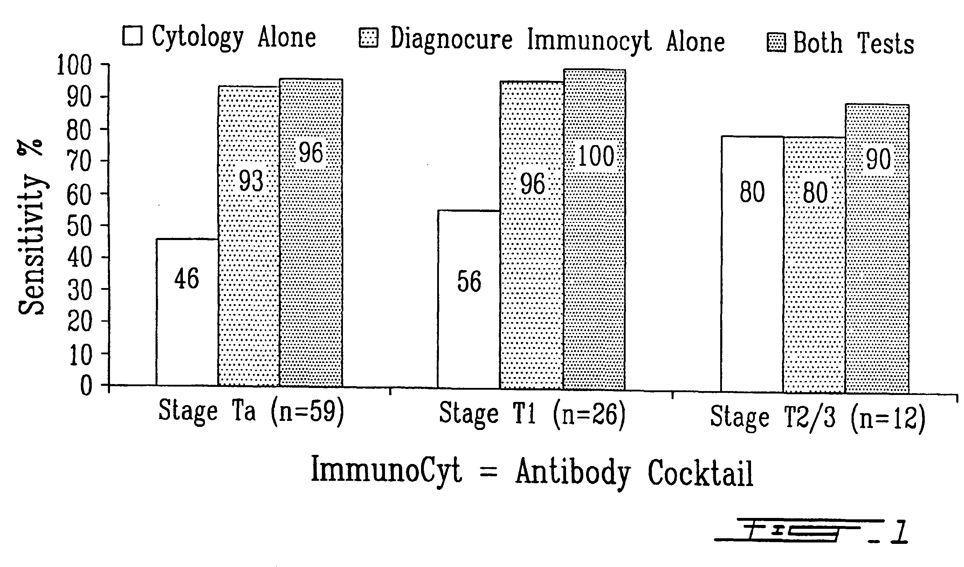

Figure 1 shows a histogram demonstrating the relative sensitivities of conventional cytology, of the M344, 19A211 and LDQ10 immunological composition and, of both, in detecting bladder cancer at different stages thereof.
Other objects, advantages and features of the present invention will become more apparent upon reading of the following non-restrictive description of preferred embodiments with reference to the accompanying drawing which is exemplary and should not be interpreted as limiting the scope of the present invention.

### DETAILED DESCRIPTION OF THE INVENTION

Prior to achieving the preferred immunological composition of the invention, the contribution of mAb M344 and LDQ10 to an immunocytological test for bladder cancer assessment was evaluated.

Briefly, cells were prepared by a method adapted from that described in great details below (see Example 1 to Example 12). The two mAbs were detected with different fluorochromes so as to distinguish their reactivity with the cells. In the following two tables M344 was detected by its fluorescein fluorescence while LDQ10 was detected with Texas Red^{®} fluorescence. The cells were collected and fixed as described below. Briefly, the cell preparation method is a modified Papanicolaou method which combines the standard cell staining method of Papanicolaou with the immunoflurorescence staining. This novel combination permits a validation that the observed fluorescence is on the cells and is not artifactual. The modified Papanicolaou staining method is described in detail in Example 5.

Table 1 shows that the percentage of bladder cancer cells TMGHU-3 (a low-grade bladder cancer cell line grown as a tumor in nude mice) recognized by the M344 mAb varies widely (between 30-60%). The LDQ10 mAb however, is much more constant in the number of recognized cells.

**Table 1**

| | Exp. | | Exp. | | Exp. | | Average % of 3 Exp. |
|---|---|---|---|---|---|---|---|
| Lot of cells #1 | 1 | % | 2 | % | 3 | % | |
| Nb. positive cells | | | | | | | |
| M344 | 66 | 65 | 58 | 55 | 71 | 61 | 60 |
| LDQ10 | 86 | 84 | 74 | 70 | 93 | 80 | 78 |
| LDQ10 + M344 | 64 | 63 | 51 | 49 | 77 | 66 | 59 |
| Total fluorescent cells | 88 | 86 | 75 | 71 | 91 | 78 | 78 |
| Total Nb. of cells counted | 102 | 100 | 105 | 100 | 116 | 100 | |
| | | | | | | | |

| | Exp. | | Exp. | | Exp. | | Average % of 3 Exp. |
|---|---|---|---|---|---|---|---|
| Lot of cells #2 | 1 | % | 2 | % | 3 | % | |
| Nb. positive cells | | | | | | | |
| M344 | 55 | 33 | 34 | 34 | 41 | 33 | 33 |
| LDQ10 | 87 | 52 | 68 | 68 | 77 | 63 | 61 |
| LDQ10 + M344 | 39 | 23 | 29 | 29 | 34 | 28 | 27 |
| Total fluorescent cells | n/a | | n/a | | 87 | 71 | 71 |
| Total Nb. of cells counted | 167 | 100 | 101 | 100 | 123 | 100 | 100 |

It should be recognized that the number of positive cells counted for either of M344 and LDQ10 also includes a fraction of doubly labeled cells. Hence, the discrepancy observed if one compares the total of positive cells labeled with only M344 or LDQ10 with the total number of positive cells. Strikingly, under this particular set of conditions using the TMGHU-3 cells, the contribution of M344 to the overall sensitivity is not apparent (i.e. lot #1 exp. 3 compare positive cells with M344 (71), LDQ10 (93) vs. the total number of positive cells (91). It appears however that a patient's tumor cells which are not glycosylated and hence not recognized by M344, might be recognized by LDQ10 and the degree of glycosylation might vary from patient to patient. This hypothesis needed to be tested with other cells. The results of such an experiment are presented in Table 2 which shows the numbers of cells uniquely reacting with a particular mAb, and reacting with both (double labelling M344-FITC and LDQ10).

**Table 2**

| | Total cell count | Nb. of urotheliales cells | Nb. of positive M344 cells only | Nb. of positive LDQ10 cells only | Nb. of positive M344 and LDQ10 cells | Total positive cells |
|---|---|---|---|---|---|---|
| 1 | | | 51 | 0 | 4 | 55 |
| 2 | | | 233 | 397 | 500 | 1130 |
| 3 | 5076 | 1800 | 76 | 219 | 432 | 727 |
| 4 | | | 19 | 5 | 13 | 37 |

By comparing for example the number of positive cells for M344 to that for LDQ10 and to the number of doubly labeled cells, it becomes clear that a significant number of cells are recognized by LDQ10 but not by M344 (the reverse also being true). It follows that a combination of these two antibodies could increase the sensitivity of a bladder cancer detection test. The results obtained using M344 and LDQ10 mAbs and the variability in their contribution prompted an analysis of further markers which could be used to further increase the sensitivity of the immunocytological test. mAb 19A211 was surprisingly found to significantly contribute to the aforementioned sensitivity.

The performance characteristics of the immunological composition comprising monoclonal M344 (ATCC HB 9678), 19A211 (ATCC HB 9679) and LDQ10 (ATCC HB 12417) (hereinafter referred to as the antibody cocktail) on bladder cancer was formally assessed.

The study involved 102 asymptomatic controls, and 198 patients with confirmed bladder cancer. Combining this test with conventional urinary cytology improves accuracy. Depending on the cut off level used in the interpretation of a positive result, the highest sensitivity of the test was 95%, with a corresponding specificity of 76.5%. Thus, the immunological composition offers the possibility of non-invasive diagnosis and monitoring of bladder cancer, with acceptable performance characteristics.

### Methods

### Study population

The study was performed in 17 centres in Ontario, Quebec, and the Atlantic provinces, during 1996 and 1997. Bladder cancer patients were diagnosed on the basis of abnormal cystoscopy, with some being newly diagnosed cancers, but the majority being recurrent tumors. Biopsy results were available for some, but not all of the cancer patients. Cytology results done by the treating clinician were not used in this study. Volunteers were healthy, asymptomatic men and women, with no known urinary pathology. Smoking status of patients and volunteers was not recorded.

Ethical approval for the study was obtained from individual institutions. Informed consent was obtained from all patients, and is on file.

### Sampling procedure

Voided urine samples (20 to 40 mL) were collected from all subjects, and were immediately fixed with an equal volume of 50% ethanol in water (in the bladder cancer patients, voided urine was obtained before cystoscopy). The samples were stored at 4° C until being shipped for blinded laboratory analysis, within five days of collection. Upon receipt of the sample, 1 mL of fixative solution (see below) was added, and the sample incubated at room temperature for an hour. Cells were collected by filtration through a membrane filter connected to a vacuum pump and filters were then rinsed with 3 mL of Saccomano solution. The cells were then blotted on two consecutive Silanized microscope slides and immediately fixed with Cytoprep^{™} (which fixes and stabilizes the cells on the slides while maintaining their morphological characteristics). The first slide was used for the fluorescence staining with the antibody cocktail, and the second slide was stained by standard Papanicolaou technique for routine cytology.

The first slides were first fixed with a modified Papanicoulaou staining, using consecutive incubation in hematoxylin, differentiator, stain OG-6, and stain EA-65 solutions (see below for further details). After rehydration in demineralized water, the cells were then incubated with 150 µL of blocking solution (see below) for 20 minutes at room temperature in a closed humid chamber. The slides were then drained of the blocking solution and incubated with the antibody cocktail labeled fluorescent markers for an hour at room temperature. Briefly, M344 and LDQ10 were labeled with Fluorescein white 19A211 was labeled with Texas Red^{®} according to conventional methods. The slides were then rinsed in PBS containing 0.05% Tween^{®} 20, and mounted with a cover slip.

### Sample analysis

The total number of cells on a slide served as a quality control measure, with any slide containing less than 500 cells being rejected. (In practice, a rejected sample should lead to the collection being repeated). The slides were read under an epifluorescence microscope. Red fluorescence showed a cell positive for high-molecular-weight glycosylated CEA, and green fluorescence showed a cell positive for bladder cancer mucins. Samples were termed positive if they showed one or more green fluorescent cells. Varying cut off levels of red fluorescent cells, between one and ten, were evaluated as positivity levels, to assess the impact on sensitivity and specificity. Specimens were also termed positive if urinary cytology was positive.

For the control group, assumed not to have cancer, specificity was calculated as the number of negative samples divided by the total number of samples from that group. For the bladder cancer patients, sensitivity was determined by dividing the number of positive samples by the total number of samples from the group. Subgroup analyses were conducted based on established stage and grade of bladder cancer, for those patients for whom the information was available.

### Results

The control group was made up of 102 asymptomatic volunteers, half of whom were male. Most of the control group members were over 50 years of age. Among the control group, the rate of rejection of samples because of inadequate numbers of cells was 17.9%. Of the bladder cancer patients, rejection was necessary in 5.2%. In the samples analysed by cytology, the corresponding rejection rates were 11.1% among normals, and 2.2% among bladder cancer patients.

Table 3 shows the sensitivity (based on 198 patients with cancer), and the specificity (based on 102 control patients), for various combinations of immunofluorescent and cytology results. Samples were called positive for cancer if any one of the tests used was positive. Out of the 198 bladder cancer patients tested, 77 specimens were found positive for antibody cocktail alone, thus, 77 patients would have been missed by cytology, on the other hand, only five specimens were found positive for cytology alone, therefore, the antibody cocktail would have only missed 5 patients.

**Table 3: Sensitivity and specificity of antibody cocktail and cytology, using different combinations and cut-off values**

| Cytology used | Green fluorescence: one or more cells positive | Red fluorescence: cut off values for positivity | Sensitivity (%) | Specificity (%) |
|---|---|---|---|---|
| Yes | --- | --- | 44.9 | 100 |
| No | + | --- | 64.0 | 95.3 |
| No | --- | 1 cell cut-off | 88.8 | 77.3 |
| No | --- | 5 cell cut-off | 74.4 | 91.0 |
| No | --- | 10 cell cut-off | 64.8 | 95.3 |
| No | + | 1 cell cut-off | 90.4 | 76.5 |
| No | + | 5 cell cut-off | 80.8 | 89.8 |
| No | + | 10 cell cut-off | 76.0 | 94.9 |
| Yes | + | 1 cell cut-off | 95.2 | 76.5 |
| Yes | + | 5 cell cut-off | 88.8 | 89.8 |
| Yes | + | 10 cell cut-off | 84.0 | 94.9 |

In Table 4, the sensitivity of the antibody cocktail, with or without cytology, is shown for patients in whom the pathological stage of bladder cancer was known from biopsy (97 of 198 patients).

**Table 4: Sensitivity (%) of antibody cocktail and cytology, using different combinations and cut-off values, for different stages of bladder cancer (note that green fluorescence was used as an additional positivity criterion whenever red fluorescence was used)**

| Tumor stage | Cytology used with antibody cocktail | 1 cell cut-off for red fluorescence | 5 cell cut-off for red fluorescence | 10 cell cut-off for red fluorescence |
|---|---|---|---|---|
| Ta (n=59) | No | 92.9 | 82.5 | 77.2 |
| Ta | Yes | 96.4 | 89.5 | 84.2 |
| T1 (n=26) | No | 96.0 | 88.0 | 84.0 |
| | Yes | 100.0 | 92.0 | 92.0 |
| T2/3 (n=12) | No | 80.0 | 70.0 | 70.0 |
| T2/3 | Yes | 90.0 | 90.0 | 90.0 |

Figure 1 shows a comparison between the sensitivity of cytology alone, versus the antibody cocktail (using one cell red plus green fluorescence cutoff), and the combination thereof. Note that the sensitivity of the antibody cocktail was higher than cytology alone for superficial cancers, but the combination of both was even more sensitive. The antibody cocktail plus cytology accurately detected 100% of T1 tumors, and 96.4% of Ta cancers.

A similar analysis is shown in Table 5, this time based on the histological grade of bladder cancer. Cytology alone detected 29.6% of grade 1 cancers, 51.1% of grade 2, and 76% of grade 3. The antibody cocktail showed higher sensitivity than cytology alone at all grades, and again the combination gave better sensitivity than either test alone.

**Table 5: Sensitivity (%) of antibody cocktail and cytology, using different combinations and cut-off values, for different grades of bladder cancer**

| Tumor grade | Cytology used with antibody cocktail | 1 cell cut-off for red fluorescence, and / or 1 cell green fluorescence | 5 cell cut-off for red fluorescence, and / or 1 cell green fluorescence | 10 cell cut-off for red fluorescence, and / or green fluorescence |
|---|---|---|---|---|
| grade 1(n=27) | No | 85.2 | 74.0 | 66.6 |
| grade 1 | Yes | 88.8 | 81.5 | 74.0 |
| grade 2(n=43) | No | 93.0 | 90.6 | 88.3 |
| grade 2 | Yes | 97.3 | 95.3 | 93.0 |
| grade 3(n=25) | No | 96.0 | 92.0 | 84.0 |
| grade 3 | Yes | 100 | 100 | 96.0 |

### Discussion

The performance of an antibody cocktail test for bladder cancer was examined. In this particular embodiment, three different monoclonal antibodies to detect bladder cancer were used. One of the antibodies is to a form of CEA, an antigen found in many human cancers. The other two monoclonal antibodies are directed against mucins, which are elaborated by most bladder cancers, but not by normal urothelial cells (Bergeron et al, 1996, J Biol Chem 271:6933-9). The test is performed in the laboratory, using standard laboratory equipment such as a fluorescence and filtration device. Training in the preparation and interpretation of the antibody cocktail test is aided by the use of standard positive and negative control slides. For this study, three cytotechnicians were trained: no formal measurement of interrater reliability was conducted.

The performance characteristics of the antibody cocktail were compared with those of conventional urinary cytology. The sensitivity of urinary cytology in the study was 44.9%, higher than the rate of 30% normally found. The improvement can be attributed at least, in part, to improved conservation of morphological features due to the fixative used, the fixation method, the improved recovery of cells from the filtration procedure and the choice of two different labels for the mabs of the cocktail. In itself, the antibody cocktail of the present invention therefore provides a very significant advantage as compared to conventional cytotology.

A limitation of this study is that no diagnostic cystoscopy was done in the control group. Although none of the group had significant symptoms, it is possible that some of them had occult bladder cancer. However, the probability of an asymptomatic volunteer having a bladder cancer is lower than one percent (given the annual incidence of bladder cancer in Canada), and this would not influence results in a meaningful way.

Several characteristics of the antibody cocktail-based test bear discussion. As can be seen in Table 3, decreasing the cut off value for the number of red fluorescent cells defined as a positive result consistently increased the sensitivity (fewer false negatives were found), but decreased specificity (more samples were falsely called positive). Using both types of fluorescence increased sensitivity over either type alone, while again decreasing specificity. The addition of cytology increased sensitivity, with no adverse effect on specificity.

The sensitivity of the antibody cocktail-based test was highest for superficial bladder cancer, while cytology was more sensitive with infiltrating cancers. Conversely, the sensitivity of the antibody cocktail-based test increased with increasing grade of bladder cancer. The combination of the antibody cocktail-based test and cytology offered 88.8% to 100% sensitivity for all stages, and all grades of bladder cancer in this study. The diagnostic accuracy of antibody cocktail in the setting of noncancerous lower urinary tract pathologies such as infections or BPH is currently under investigation.

High sensitivity is the most important characteristic of a diagnostic test (more so than the specificity), in patients suspected of having bladder cancer on the basis of symptoms or history. Indeed, these patients will undergo cystoscopy unless a test can exclude the presence of a tumor, a goal that is reached with a highly sensitive test.

To reiterate, the test must aim for the best sensitivity possible 90%-100%, even if specificity is compromised. Preferably, no false negatives are obtained even if that translates in the obtention of false positives. The antibody cocktail-based test of the present invention therefore provides a significant improvement in sensitivity over the known non-invasive tests that have been examined for use in this clinical situation which repeat overall sensitivities of 50%-70% (Sarowsdy et al, 1995, J. Urol., 154:379-83; Soloway et al., 1996, J. Urol., 156:363-7).

The detection and monitoring of bladder cancer has implications not only for clinical management, but also for health care economics. A non-invasive test that is highly sensitive may provide improved clinical information while also allowing for optimal allocation of financial resources.

### Conclusions

The present invention demonstrates that the antibody cocktail-based test has excellent sensitivity in the diagnosis of superficial bladder cancer. Given the non-invasive nature of the test, patient acceptance is expected to be high. The combination of the antibody cocktail-based test and cytology further provides a significant sensitivity for more invasive types of bladder cancer. The present invention therefore provides a simple, non-invasive and sensitive method of bladder cancer detection in general. Furthermore, the antibody cocktail-based test offers substantially higher sensitivity when compared with conventional urinary cytology, while preserving the advantage of the high specificity of cytology. As a diagnostic and monitoring test, the antibody cocktail-based test offers distinct advantages over either of the currently used testing procedures.

The present invention is described in further detail in the following non-limiting examples.

### EXAMPLE 1

### ANTIBODY COCKTAIL TEST FOR THE DETECTION OF BLADDER CANCER

### Introduction

In a preferred embodiment, the antibody cocktail test is an immunocytofluorescent technique based on a monoclonal antibody cocktail labeled with fluorescent markers. It has been shown to be highly sensitive for the detection of superficial bladder cancer cells. The antibody cocktail consists of two antibodies which bind to a mucin specific to bladder cancer and an antibody specific to a glycosylated form of carcinoembryonic antigen (CEA). These antigens are expressed on superficial bladder tumor cells. The cocktail is then incubated with fixed urinary cells which have been transferred onto a microscope slide after being harvested by filtration. Cells presenting tumor specific antigens are detected by epifluorescence microscopy.

### Reagents

1. Blocking Solution: solution of buffered diluted goat serum containing Proclin^{®} (antibacterial substance), and ready-to-use for up to 100 tests. More specifically, the blotting solution contains:
   - PBS 1 x pH 7.3
   - 5% goat serum
   - 0.1 Proclin^{™}
   This solution is used for the blocking of non specific sites, before fluorescent labelling. The blocking solution is presented in a dropper bottle, and contains 15 mL of solution. The dropper bottle delivers ~50 µL per drop. Store at 4°C.
2. Antibody Cocktail: solution of three fluorescent labeled monoclonal antibodies, containing Proclin^{®}, and ready-to-use for 100 tests. More specifically, the antibody cocktail is in a solution containing:
   - PBS 3x
   - 0.05M KF
   - 1% goat serum
   - 0.1 Proclin^{™}
   The anti-Mucin antibodies are labeled with Fluorescein (green light emission; M344 and LDQ10), the anti-glycosylated-CEA antibody is labeled with Texas Red^{®} (red light emission; 19A211). The antibody cocktail is presented in a dropper bottle containing 15 mL of solution. The dropper bottle delivers -50 µL per drop. Store at 4°C.
3. Sample Fixative: a powder containing buffer and polyethylene glycol (PEG). The powder is presented in two sealed packets. The content of one packet is sufficient for 50 samples. This powder is dissolved with demineralised water to provide 50 mL per packet and is used to fix urinary cells. More specifically, the packet to prepare the fixative contains:
   - 1.96 g NaCl
   - 3.78 g MOPSO
   - 20 g PEG-4000
   After dissolution, store at 4°C for up to 3 months.
4. 50% ethanol in water used at the collection site
5. Phosphate buffered saline solutions (PBS) pH 7.4 ± 0.2 (see Example 2)
6. Phosphate buffered saline solution (PBS) pH 7.4 ± 0.2 containing 0.05% Tween^{®} 20 (see Example 2)
7. Mowiol slide mounting medium (see Example 3)
8. Saccomano solution (see Example 4)
9. Colorants, ethanol and toluene solutions for Papanicolaou staining (see Example 5)
10. Sulfochromic acid (see Example 5)

### Materials

1. Demineralised water
2. Absorbent tissue (ex. Kimwipes^{®}, Kimberley-Clark)
3. Epifluorescence microscope fitted with dichroic mirrors and filters for Fluorescein and Texas Red^{®} emission light detection (ex. Omega XF53) and a mercury lamp 100 W, 12 V
4. Filtration device composed of a filter adapter o 25 mm (ex. Swinnex^{®}), 60 mL syringe, vacuum chamber (ex. Vac-Elut^{®} 10, Varian), needle valve syringe adapters (ex. Varian), trap for liquid, and vacuum pump.
5. Polycarbonate membrane filters φ 25 mm, 8 µm porosity (ex. VWR cat. # 28158-806)
6. Forceps
7. Humid chamber for slides
8. Baths for the different steps of the Papanicolaou staining and silanisation of slides
9. Pasteur pipettes
10. Silanized microscope slides (see Example 5)
11. Cover slips
12. Spray fixative of cells on slide (ex. Cytoprep, Fisher cat. # 12-570-10)
13. Slide holders
14. Wash bottle
15. 80 mL containers for urine samples

### Preparation of Fixative

In a container, dissolve the powder from the fixative package with demineralised water in order to make 50 mL of fixative (approximately 34 mL of demineralised water). Stir the solution until all the powder is completely dissolved. The solution can be heated to 37°C in a water bath to facilitate the dissolution.

### EXAMPLE 2

### PREPARATION OF PBS SOLUTIONS

### Reagents

Di-sodium hydrogen phosphate anhydrous (Na₂HPO₄): M.Wt. 141.96
Potassium chloride (KCl): M.Wt. 74.55
Potassium phosphate di-hydrogen anhydrous (KH₂PO₄): M.Wt. 136.09
Sodium chloride (NaCl): M.Wt. 58.44
Polyoxyethylenesorbitan monolaurate (ex. Tween^{®} 20)
Sodium hydroxyde solution (NaOH) 10 N
Hydrochloric acid (HCl) 10 N

### Preparation of PBS 10X

1. Weigh

| | |
|---|---|
| 45.3 g | Na₂HPO₄ |
| 8.0 g | KCI |
| 8.0 g | KH₂PO₄ |
| 300.0 | g NaCl |

2. Place the ingredients in a container capable of holding more than 4.0 L. Add 3.5 L of demineralised water and dissolve the ingredients completely.
3. Adjust the pH to 7.4 ± 0.2 with 10 N NaOH or 10 N HCl.
4. Add demineralised water to complete to 4.0 L.
5. Label and date the solution. PBS 10X can be stored up to 1 month at room temperature.

### Preparation of PBS 1X

1. Place 400 mL of PBS 10X in a container capable of holding more than 4.0 L.
2. Add 3.0 L of deionised water and mix thoroughly.
3. Adjust the pH to 7.4 ± 0.2 with 10 N NaOH or 10 N HCl.
4. Complete the volume to 4.0 L with demineralised water.
5. Label and date the solution. PBS 1X solution can be kept at 4°C, for up to one week.

### Preparation of PBS/Tween® 20 (0.05%)

1. Add 2 mL of Tween^{®} 20 to 4.0 L of PBS 1X.
2. Thoroughly mix the solution.
3. Label and date the solution. PBS/Tween^{®} solution can be kept at 4°C, for up to one week.

### EXAMPLE 3

### MOUNTING MEDIUM MOWIOL

### Reagents

1. Mowiol 4-88 (Calbiochem, cat. # 475904): store at room temperature.
2. Trishydroxymethyl aminomethane (Tris)
3. Hydrochloric acid (HCl) 1 N
4. Glycerol

### Preparation of Tris HCl buffer

1. Weigh
   24.2g Tris
2. Place the Tris in a container capable of holding 1.0L and add 0.75 L of demineralised water.
3. Adjust the pH to 8.5 ± 0.2 with 1 N HCl.
4. Complete the volume to 1.0 L with demineralised water.
5. Label and date the solution. Tris buffer can be stored for 1 month at 4°C.

### Preparation of a Mowiol solution

1. Weigh
   7.2 g Mowiol 4-88
   18.0g glycerol
   This procedure should be carried out in a flow hood.
2. Mix Mowiol and glycerol in a glass container capable of holding 100 mL. Add 18 mL of demineralised water and leave over night to dissolve the Mowiol.
3. Add 36 mL of Tris/HCl buffer.
4. Heat the mixture to 50°C in a water bath, until the liquid is clear (about two hours).
5. Centrifuge for 15 minutes at 5000 x *g* to remove any debris or crystals.
6. Distribute the Mowiol solution in 1.5 mL aliquots and label these with the date of production. Store these at -20°C.
7. A working aliquot can be kept at room temperature, for up to 7 days.
Ensure that the Mowiol solution is at room temperature before use.

### EXAMPLE 4

### PREPARATION OF SACCOMANO SOLUTION

### Reagents

1. 50% ethanol (100 mL 99% ethanol and enough demineralised water to obtain 200 mL)
2. Polyethylene glycol (PEG), M.Wt. 1500

### Procedure

1. Pour 200 mL of 50% ethanol in a container.
2. Place this container on a balance, and tare.
3. Melt the PEG 1500 in a microwave oven.
4. Pipette the PEG solution, and add 4.0 g of PEG to the container. Mix thoroughly the PEG and ethanol.
5. The Saccomano solution can be kept at 4°C, for up to one month.

### EXAMPLE 5

### MODIFIED PAPANICOLAOU STAINING FOR ANTIBODY COCKTAIL

### Materials

1. Differentiator (394 mL 70% ethanol and 6 mL NH₄OH)
2. Hematoxylin (Harris) without acetic acid (ex. VWR, cat. # R03312-82)
3. Papanicolaou stain EA-65 (ex. VWR, cat. # R03315-82)
4. Papanicolaou stain OG-6 (ex. VWR, cat. # R03422-82)
5. 95% ethanol
6. 99% ethanol (absolute ethanol)
7. 80% ethanol (3.2 L 99% ethanol to 4.0 L with demineralised water)
8. 70% ethanol (2.8 L 99% ethanol to 4.0 L with demineralised water)
9. 50% ethanol (2.0 L 99% ethanol to 4.0 L with demineralised water)
10. 50% ethanol/toluene (200 mL of 99% ethanol and 200 mL with toluene)

### Procedure

1. Place the dried sample slides in a slide holder.
   NOTE: Each of the following steps (2 to 29) requires a bath specific to this step.
2. Immerse in 80% ethanol bath (10 times).
3. Immerse in 70% ethanol bath (10 times).
4. Immerse in 50% ethanol bath (10 times).
5. Immerse in demineralised water bath (3 times).
6. Immerse in hematoxylin bath for 45 seconds.
7. Immerse in demineralised water bath (10 times).
8. Immerse in demineralised water bath (10 times).
9. Immerse in demineralised water bath (10 times).
10. Immerse in 50% ethanol bath (10 times).
11. Immerse in the differentiator bath for 45 seconds in the case of the antibody cocktail or 75 seconds for the standard Papanicolaou staining.
12. Immerse in 70% ethanol bath (10 times).
13. Immerse in 80% ethanol bath (10 times).
14. Immerse in 95% ethanol bath (10 times).
15. Immerse in OG-6 bath for 75 seconds.
16. Immerse in 95% ethanol bath (10 times).
17. Immerse in 95% ethanol bath (10 times).
18. Immerse in 95% ethanol bath (10 times).
19. Immerse in EA-65 bath for 150 seconds.
20. Immerse in 95% ethanol bath (10 times).
21. Immerse in 95% ethanol bath (10 times).
22. Immerse in 95% ethanol bath (10 times).
23. Immerse in 95% ethanol bath (10 times).
   Stop here the treatment for the antibody cocktail, and immerse the slides in demineralised water.
   If you are performing the standard Papanicolaou staining, pursue with the following steps.
24. Immerse in 99% ethanol bath (10 times).
25. Immerse in 99% ethanol bath (10 times).
26. Immerse in 50% ethanol/toluene bath (10 times).
27. Immerse in toluene bath (10 times).
28. Immerse in toluene bath (10 times).
29. Immerse in toluene bath (10 times).
30. Mount the slide with a cover slip using mounting medium Entallan^{®} (ex. VWR, cat. # M07961-70).
   All baths used in this procedure should be changed at least once a week. Wash baths (steps 7, 16, and 20) should be changed after each use.

### EXAMPLE 6

### PREPARATION OF SILANIZED SLIDES

### (PROCEDURE FOR 600 SLIDES)

Note: There are available precleaned slides (ex. VWR, cat # 48312-705). If you use them, proceed directly to the silanisation of the slides.

### Slide Cleaning

### Materials

1. Microscope slides with 1 end frosted (ex. Goldline^{®}, VWR, cat. # 48323-185 or equivalent)
2. Chromerge^{®} (contains chromic acid) (ex. VWR, cat. #21865-000)
3. Sulfuric acid (H₂SO₄) 18 N, 4.25 kg bottle

### Procedure

Steps 1 to 3 must be carried out under a fumehood.
1. Slowly add 25 mL of Chromerge^{®} to 4.25 Kg of concentrated sulfuric acid to form sulfochromic acid.
2. Place approximately 400 mL of sulfochromic acid in a glass container.
3. Place the slides in a slide holder and immerse them for 5 minutes in the sulfochromic acid.
4. Rinse the acid-treated slides with tap water for at least 5 minutes to eliminate all traces of sulfochromic acid.
5. Immerse the slides 10 times in a demineralised water bath.

### Silanisation of the Slides

### Materials

1. Acetone
2. 3-aminopropyltriethoxysilane (AAS)

### Procedure

1. Add 16 mL of 3-aminopropyltriethoxysilane to 784 mL of acetone to obtain a 2% v/v AAS.
2. Prepare 2 baths containing 400 mL of 2% v/v AAS; 2 baths of 400 mL acetone; 2 baths of 400 mL demineralised water.
3. immerse the slides 4 times in the first AAS bath. Remove the slide holder and leave the excess liquid to drain.
4. Immerse the slides 4 times in the second ASS bath. Remove the slide holder and allow the excess liquid to drain.
5. Immerse the slides 2 times in the first acetone bath for 2 seconds per immersion. Remove the slide holder and allow the excess liquid to drain.
6. Immerse the slides 2 times in the second acetone bath for 2 seconds per immersion. Remove the slide holder and allow the excess liquid to drain.
7. Immerse the slides 2 times in the first deionised water bath for 2 seconds per immersion. Remove the slide holder and allow the excess liquid to drain.
8. Immerse the slides 2 times in the second deionised water bath for 2 seconds per immersion. Remove the slide holder and allow the excess liquid to drain.
9. Dry the treated slides at 37°C over night.
10. The treated slides can be stored for up to 6 months at room temperature. They must be protected from dust.

### EXAMPLE 7

### URINARY SAMPLE COLLECTION AND TREATMENT

### At the collection site

It is preferable not to collect first morning micturation, since the biological sample may be of a lesser quality. It is recommended to use urine samples taken during the day. It is not necessary to take samples from fasting patients.

Immediately after collection place 20 to 40 mL of urine in a 80 mL urinary cytology container. Add an equal volume of 50% ethanol (ex. 1.0 L 99% ethanol and the volume of demineralised water necessary to complete to 2.0 L) to the sample. The container is firmly closed and the contents thoroughly mixed.

Send the samples immediately to the laboratory.

### At the laboratory

It is recommended to perform the antibody cocktail procedure on the samples within 7 days from collection, once cells have been transferred to slides. The samples must have been stored at 4°C.

Upon reception, add 1 mL of fixative solution to the urine/ethanol mixture. The container is firmly closed and thoroughly mixed to disperse the fixative. The fixed samples must be incubated at room temperature for 1 hour before proceeding with the filtration of samples.

### EXAMPLE 8

### FILTRATION OF SAMPLES

1. Mark two silanized slides per sample (see Example 6). All samples and reagents should be brought to room temperature before proceeding with the preparation to ensure the best results.
2. Place a membrane filter in the Swinnex^{®} filter holder. Ensure that the shiny side of the membrane filter is upward so that this side will be in contact with the urine.
3. Install the filtration system (syringe, Swinnex^{®}, adapter, and Vac-Elut apparatus) and connect with the vacuum pump. Ensure that there is a trap between the Vac-Elut^{®} and the pump.
4. Vigorously mix the sample by hitting the container 3 to 4 times against a working surface. The urine samples must be vigorously mixed before filtration to break up any mucus present in some samples and resuspend cells which may have settled or stick to the container.
5. Fill the syringe with 60 mL of urine/ethanol/fixative sample, and apply the vacuum.
6. Add the rest of the sample if the flow through the filter is unrestricted.
7. Stop the filtration procedure by switching the pump off once the urine flow is restricted or the sample is almost completely filtered. Ensure that a small amount of urine stays in the syringe to prevent the filter from drying out. It is absolutely necessary to avoid drying of the cells during the different filtration and staining steps, to avoid modifications to the cellular morphology.
   It is recommended not to force the filtration as this will result in too many cells on the filter.
   Recover any unfiltered urine in a container by disconnecting the syringe with its stand (Swinnex^{®}) from the Vac-Elut^{®}, and emptying the combined syringe-stand. This sample can be stored at 4°C, for up to 7 days from time of collection.
8. After urine filtration, add 3 mL of Saccomano solution to the syringe, and then apply a vacuum to rinse the membrane filter. Do not allow all the Saccomano solution to pass but keep a small amount in the syringe to prevent the filter from drying out. Wait 1 minute.
If it is not possible to wait one minute because all the Saccomano solution may be filtered through. Proceed to the cell transfer immediately.

### EXAMPLE 9

### CELL TRANSFER TO SUDES (TWO SLIDES FOR ONE SAMPLE)

Cell transfer is performed on the 2 slides previously labeled.
1. Place a drop of Saccomano solution on slide 2 in order to wet the filter and ease the cell content transfer to this slide (see step 5).
2. Remove the Syringe and the Swinnex^{®} filter adapter from the filtration apparatus and recover the filter from the Swinnex^{®} using forceps.
   The following steps should be executed as quickly as possible to avoid drying of the filter.
3. Place the filter onto slide 1, so that the filter surface which was in contact with the sample is against the slide.
4. Place absorbent tissue wetted with PBS 1X on the filter and transfer the filter content by applying a firm pressure from the hand's edge. Make sure that the whole filter surface is well pressed.
5. Remove the absorbent tissue. Carefully remove the filter from slide 1. Immediately place the filter, sample side against the slide, onto slide 2. Before continuing with slide 2, immediately fix slide 1 by spraying it with Cytoprep^{™} and leave the slide to dry at room temperature.
6. On slide 2, repeat step 4. Remove the absorbent tissue. Carefully remove and discard the filter. Immediately spray slide 2 with Cytoprep^{™} and leave the slide to dry at room temperature.
7. It is recommended to proceed immediately to the next section (see Example 10). Should this not be possible, the slides can be stored for up to 3 days at -20°C, protected from light and dust. Slides dedicated to cytology can be stored at room temperature for up to 6 months.

### EXAMPLE 10

### ANTIBODY COCKTAIL

### Coupling of antibodies

The monoclonal antibodies were dialyzed overnight at 4°C in a phosphate buffer (K₂HPO₄ + NaH₂PO₄) 100 mM + 150 mM NaCl pH 8.3. the mabs were measured by reading the O.D. at 280 nm following dialysis. The coupling reaction was performed in the above-mentioned phosphate buffer with a mAb oncentration of 5 mg/ml. The fluorochrome succinimydil ester was dissolved in DMSO. For the different antibodies, the initial molar ratio of fluorochrome to antibody was:

| | |
|---|---|
| LDQ10 | 7:1 |
| M344 | 10:1 |
| 19A211 | 5:1 |

The mab fluorochrome mixtures were incubated one hour in darkness, without shaking and then purified through a G-25 column using a elution buffer (Phosphate buffer 100 mM - NaCl 150 mM, EDTA 5mM, pH 7.3)

### Modified Papanicolaou staining (slides 1)

IMPORTANT: Modified Papanicolaou staining (see Example 5) is an essential step before proceeding with the antibody cocktail procedure.
1. Before treating the slides with the antibody cocktail procedure, they are stained using a modified Papanicolaou staining. It is essential to respect the times of treatment in the following staining baths:

| | |
|---|---|
| hematoxylin | 45 seconds |
| differentiator | 45 seconds |
| stain OG-6 | 75 seconds |
| stain EA-65 | 150 seconds |

2. Follow the steps of the modified Papanicolaou staining up to the last 95% ethanol bath (step 23, Example 5).
3. Place the slides in demineralised water to rehydrate the samples and proceed immediately to the antibody cocktail procedure.

### Antibody cocktail Procedure

Ensure that PBS 1X and PBS/Tween^{®} are at room temperature before continuing with the antibody cocktail procedure.
1. Place the slides labeled 1 in a humid chamber.
2. Place 3 drops of blocking solution (∼150 µL) on the cell zone of slide 1 and incubate for 20 ± 5 minutes at room temperature in a closed humid chamber.
3. Drain the slides by placing the slide edge against absorbent paper. Do not allow the slide to dry out.
   It is necessary to ensure the maximal removal of the blocking solution before the incubation with the antibody cocktail, to avoid a decrease in the fluorescence signal.
4. Place 3 drops of the antibody cocktail (~150 µL) on the cell zone and incubate for 60 ± 5 minutes at room temperature in a humid chamber. Protect the slides from light for this step and the following ones.
5. Rinse the slides with PBS containing 0.05% Tween^{®} 20 (PBS/Tween^{®}) (see Example 2) using a wash bottle. Be careful not to direct the wash to the cell zone, this could result in the detachment of the cells.
6. Place the slides in a slide holder which has been put in a bath containing a PBS/Tween^{®} solution.
7. Immerse the slide holder 10 times. After the final immersion leave the slides in contact with the PBS/Tween^{®} for 2 ± 1 minutes. Follow this with 10 rapid immersions.
8. Change the solution in the PBS/Tween^{®} bath.
9. Repeat steps 7 and 8 two times.
10. Immerse the slides in a PBS 1X bath (see Example 2) 10 times. At this stage the slides can be left in PBS 1X from 1 to 60 minutes before mounting the cover slips.

### EXAMPLE 11

### COVER SLIP MOUNTING AND READING OF THE SLIDES

1. Place 20 µL of Mowiol on a cover slip. The use of Mowiol eliminates the need to seal the cover slip.
2. Place the cell zone of the slides against the cover slip. Avoid the formation of bubbles between the cover slip and the slide. To allow drying slides must be on the horizontal position for a period of 24 hours.
3. Protect the slides from light and proceed with microscope examination within 24 hours.
4. Leave the slides reach room temperature (about 15 minutes) before the reading.

### Reading of the slides

The reading of slides requires personnel trained in the use of a microscope and knowing how to identify various urine components (red blood cells, inflammatory cells, crystals, mucus, etc.). Green fluorescence emitted by positive cells is easily identified and is found on the cell membrane and in the vacuoles; green fluorescent points are considered positive. A red fluorescence emitted by positive cells will also present itself as points. In some cases, a large amount of emitted red fluorescence may not look like points but seem uniform. Some non-cancerous cells such as inflammatory cells, red blood cells or squamous cells may present a uniform orange staining. Light microscope examination may ease the discrimination of these cells. The presence of mucus may invalidate the interpretation of certain samples.
1. Use an epifluorescence microscope fitted with dichroic mirror and filters for the detection of Fluorescein and Texas Red^{®} (ex. Omega XF53 filter).
2. Rapidly scan the slide under fluorescence with a 20x objective to evaluate the uniformity of cell distribution. Should no cells be detected at this step, check for the presence of cells using light microscopy. Reject the sample should no cells be detected. Any slide which presents less than 500 cells is considered inadequate for statistical validation of a negative result in the antibody cocktail, and must be rejected. A total number of less cell than 500 can be determined when there is on average less than 1 squamous or urothelial cell (urinary cells) per microscope field with a 20x objective.
   It is essential to not overexpose the slide to light at this stage as this can result in an attenuation of fluorescence.
3. Examine the complete slide to enumerate red and/or green fluorescent emitting cells with a 20x or 40x objective.
4. If necessary, examine cells using light microscopy to improve interpretation.
5. Note the presence of crystals, red blood cells, inflammatory cells, etc.
6. After finishing the reading with fluorescence, you may evaluate the total number of cells under light microscope with a 10x objective. All slides presenting more than 500 cells can be considered as having an adequate number of cells to validate the antibody cocktail procedure and the subsequent interpretation. The total number of cells can be estimated by comparison with calibrated slides and pictures under light microscope. Reference slides with known numbers of cells may be prepared to facilitate total cell number evaluation. Trained personnel working with a 10x objective should use these reference slides to become familiarized with the total number of cells. The intervals for the total number of cells are the following:
   a) < 500 cells
   b) between 500 - 1000 cells
   c) between 1000 - 5000 cells
   d) between 5000 - 10,000 cells
   e) between 10,000 - 20,000 cells
   f) > 20,000 cells

### EXAMPLE 12

### INTERPRETATION OF RESULTS

In order to obtain a high sensitivity, while keeping a good specificity, one must establish detection cut-off values for the green and red fluorescence emitted by fluorophore-tagged antibodies. An evaluation of the antibody cocktail test was carried out with 102 samples from presumed normal volunteers, and 198 samples from patients with bladder cancer. Data was gathered to indicate the sensitivity and specificity levels using different cell cut-off values for a number of positive cells detected with red and/or green fluorescence. The specificity and sensitivity for combined red and green fluorescence results were calculated using cut off values of 1, 5 or 10 cells for the antibody giving red fluorescence, and of 1 for the antibodies giving green fluorescence.

For a cut-off value of one cell detected in red and/or green fluorescence, the antibody cocktail test combined with cytology provides a sensitivity of up to 95% for patients presenting superficial bladder cancer and a specificity of 76% for a population of presumed normal subjects. Conventional cytology alone provided a sensitivity and specificity of 44.9% and 100%, respectively.

Each laboratory should establish its own cut-off values for the detection by red and green fluorescence.

Table 6 shows an analysis of the data from samples of 97 patients for whom pathology results (biopsy) were available. The samples were classified in the following way:

| | |
|---|---|
| Stage Ta | 59 samples |
| Stage T1 | 26 samples |
| Stages T2-T3 | 12 samples |

Specificity was calculated from the samples of patients presumed normal.

**Table 6**

| | Antibody Cocktail Cell Cut-off | | | Cytology |
|---|---|---|---|---|
| | 10 | 5 | 1 | |
| Specificity | 94.9% | 89.8% | 76.5% | 100% |
| Sensitivity | | | | |
| Stage Ta | 77.2% | 82.5% | 92.9% | 45.6% |
| Stage T1 | 84% | 88% | 96% | 56% |
| Stages T2-T3 | 70% | 70% | 80% | 80% |

| Antibody Cocktail and Cytology Sensitivity | | | | |
|---|---|---|---|---|
| Stage Ta | 84.2% | 89.5% | 96.4% | |
| Stage T1 | 92% | 92% | 100% | |
| Stages T2-T3 | 90% | 90% | 90% | |

### EXAMPLE 13

### CLASSICAL CYTOLOGY

Classical cytology may be performed on the second cell transfer (slide 2). In this case proceed with the standard Papanicolaou staining (see Example 5). If there is an insufficient number of cells on slide 2 to validate standard cytology, take slide 1 after using it for the antibody cocktail test. In this case, the cover slip is removed by immersing the slide in PBS 1X within three days of performing the test. This should take 10 to 30 minutes. Proceed with the standard Papanicolaou staining with this slide (the 30 steps of Example 5). This slide can be used in routine cytology analysis.

In conclusion, the present invention discloses immunological compositions for the detection and/or prognosis of cancers expressing the targetted cancers markers of the present invention. In a particularly preferred embodiment it is shown that the detection of bladder cancer by classical cytology can be considerably improved through the concomitant use of the immunological composition comprising mAbs M344, 19A211 and LDQ10 tagged with different fluorophores, thus reaching 95% sensitivity. In any event, the present invention significantly enhances the sensitivity of bladder cancer detection as compared to the available tests which only reach 50%-70% sensitivity.

Although the present invention has been described hereinabove by way of preferred embodiments thereof, it can be modified, without departing from the spirit and nature of the subject invention as defined in the appended claims.

## Claims

1. A non-invasive, investigative test for the detection of bladder cancer comprising:
a) collecting bladder cells from a biological sample;
b) fixing said cells;
c) reacting said fixed cells with an immunological composition comprising a first mab or fragment thereof recognizing the epitopes recognized by mab M344 obtainable from the cell line having the accession number ATCC HB9678, a second mab or fragment thereof recognizing the epitope recognized by mab 19A211 obtainable from the cell line having the accession number ATCC HB9679, and a third mab or fragment thereof recognizing the epitope recognized by mab LDQ10 obtainable from the cell line having the accession number ATCC HB12417 for a sufficient time to obtain decorated cells;
d) performing an immunocytological analysis on said decorated cells; and
e) assessing the absence or presence of bladder cancer;
wherein said first and third mabs or fragment thereof are labelled with a first fluorochrome and said second mab or fragment thereof is labelled with a different fluorochrome and wherein the detection of at least one positive cell reacting with said first or third mab or fragment thereof or the detection of at least one cell reacting with said second mab or fragment thereof is considered indicative of bladder cancer.

2. The non-invasive, investigative test of claim 1, wherein said immunological composition comprises mab M344 obtainable from the cell line having the accession number ATCC HB9678, mab 19A211 obtainable from the cell line having the accession number ATCC HB9679 and mab LDQ10 obtainable from the cell line having the accession number ATCC HB12417.

3. The test of claims 1 or 2, further comprising performing classical cytological analysis on said fixed cells or said decorated cells.

4. The non-invasive, investigative test of anyone of claims 1-3, wherein the detection of at least one cell reacting with a mab recognizing an epitope recognized by mabs M344 or LDQ10 and the detection of at least one cell reacting with a mab 19A211 is considered indicative of bladder cancer.

5. The non-invasive, investigative test of anyone of claims 1-3, wherein the detection of at least one cell reacting with a mab recognizing an epitope recognized by mabs M344 or LDQ10 or the detection of at least 5 cells reacting with a mab 19A211 is considered indicative of bladder cancer.

6. The non-invasive, investigative test of anyone of claims 1-3, wherein the detection of at least one cell reacting with a mab recognizing an epitope recognized by mab M344 or LDQ10 or the detection of at least 10 cells reacting with a mab 19A211 is considered indicative of bladder cancer.

7. The non-invasive, investigative test of anyone of claims 1-3, wherein the detection of at least 5 cells reacting with a mab recognizing an epitope recognized by mabs M344, LDQ10 or 19A211 is considered indicative of bladder cancer.

8. The non-invasive, investigative test of anyone of claims 1-3, wherein the detection of at least 10 cells reacting with a mab recognizing an epitope recognized by mabs M344, LDQ10 or 19A211 is considered indicative of bladder cancer.

9. The non-invasive, investigative test of anyone of claims 1-3, wherein the sensitivity of said test is higher than 80%.

10. The non-invasive, investigative test of anyone of claims 1-3, wherein the sensitivity of said test is higher than 90%.

11. The non-invasive, investigative test of anyone of claims 1-3, wherein the sensitivity of said test is higher than 95%.

12. The non-invasive, investigative test of anyone of claims 1-3, wherein the sensitivity of said test is between 95% and 100%.

13. The test of anyone of claims 1-12, wherein said biological sample is a urine sample.

14. A method for estimating the risk of recurrence and tumor rate in bladder cancer comprising:
a) isolating bladder cells from a biological sample of a patient;
b) fixing said cells to preserve their morphological characteristics;
c) reacting said fixed cells with an immunological composition comprising a first mab or fragment thereof recognizing the epitope recognized by mab M344 obtainable from the cell line having the accession number ATCC HB9678, a second mab or fragment thereof recognizing the epitope recognized by mab 19A211 obtainable from the cell line having the accession number ATCC HB9679, and a third mab or fragment thereof recognizing the epitope recognized by mab LDQ10 obtainable from the cell line having the accession number ATCC HB12417; and
d) estimating the risk of recurrence and the tumor rate based on the reactivity of said mabs with said tumor,
wherein said first and third mabs or fragment thereof are labelled with a first fluorochrome and said second mab or fragment thereof is labelled with a different fluorochrome, and wherein said risk of recurrence of the tumor can be determined by comparing the amount of reactivity of said first mab, or fragment thereof or said third mab or fragment thereof which is associated with an increase in the tumor rate, with a reactivity of said second mab with the tumor being associated with an increase in a recurrence hazard and in recurrence rate.

15. The method of claim 14, wherein said immunological composition comprises mab M344 obtainable from the cell line having the accession number ATCC HB9678, mab 19A211 obtainable from the cell line having the accession number ATCC HB9679 and mab LDQ10 obtainable from the cell line having the accession number ATCC HB12417.

16. The method of claim 14 or 15, wherein the detection of at least one positive cell reacting with said first or third mab or the detection of at least one cell reacting with said second mab is considered indicative of bladder cancer.

17. The method of claim 14, 15 or 16 further comprising performing classical cytological analysis on said fixed or decorated cells.

18. The method of anyone of claims 14-17, wherein the detection of at least one cell reacting with a mab recognizing an epitope recognized by mabs M344 or LDQ10 and the detection of at least one cell reacting with a mab 19A211 is considered indicative of bladder cancer.

19. The method of anyone of claims 14-17, wherein the detection of at least one cell reacting with a mab recognizing an epitope recognized by mabs M344 or LDQ10 or the detection of at least 5 cells reacting with a mab 19A211 is considered indicative of bladder cancer.

20. The method of anyone of claims 14-17, wherein the detection of at least one cell reacting with a mab recognizing an epitope recognized by mab M344 or LDQ10 or the detection of at least 10 cells reacting with a mab 19A211 is considered indicative of bladder cancer.

21. The method of anyone of claims 14-17, wherein the detection of at least 5 cells reacting with a mab recognizing an epitope recognized by mabs M344, LDQ10 or 19A211 is considered indicative of bladder cancer.

22. The method of anyone of claims 14-17, wherein the detection of at least 10 cells reacting with a mab recognizing an epitope recognized by mabs M344, LDQ10 or 19A211 is considered indicative of bladder cancer.

23. The method of anyone of claims 14-17, wherein the sensitivity of said test is higher than 80%.

24. The method of anyone of claims 14-17, wherein the sensitivity of said test is higher than 90%.

25. The method of anyone of claims 14-17, wherein the sensitivity of said test is higher than 95%.

26. The method of anyone of claims 14-17, wherein the sensitivity of said test is between 95% and 100%.

27. The method of anyone of claims 14-26, wherein said biological sample is a urine sample.

28. An immunological composition for the detection of bladder cancer, comprising monoclonal antibodies (mabs) or fragment thereof recognizing antigens associated with bladder cancer wherein said mabs or fragment thereof comprise mab or fragment thereof recognizing the epitope recognized by mab M344 obtainable from the cell line having the accession number ATCC HB9678, mab 19A211 obtainable from the cell line having the accession number ATCC HB9679 and mab LDQ10 obtainable from the cell line having the accession number ATCC HB12417, wherein said mab or fragment thereof recognizing the epitope recognized by mab M344 and said mab or fragment thereof recognizing the epitope recognized by mab LDQ10 are labelled with a first fluorochrome and said mab or fragment thereof recognizing the epitope recognized by mab 19A211 is labelled with a different fluorochrome.

29. The immunological composition of claim 28, comprising mab M344 obtainable from the cell line having the accession number ATCC HB9678, mab 19A211 obtainable from the cell line having the accession number ATCC HB9679 and mab LDQ10 obtainable from the cell line having the accession number ATCC HB12417.

30. A diagnostic kit comprising the composition of claim 28 or 29.

## Patentansprüche

1. Nicht-invasiver Untersuchungstest zum Nachweis von Blasenkrebs, umfassend:
a) Sammeln von Blasenzellen ausgehend von einer biologischen Probe;
b) Fixieren der Zellen;
c) Umsetzen der fixierten Zellen mit einer immunologischen Zusammensetzung, umfassend einen ersten mAk oder ein Fragment davon, welcher bzw. welches die Epitope erkennt, die durch den mAk M344 erkannt werden, welcher von der Zelllinie mit der Hinterlegungsnummer ATCC HB9678 erhalten werden kann, einen zweiten mAk oder ein Fragment davon, welcher bzw. welches das Epitop erkennt, das durch den mAk 19A211 erkannt wird, welcher von der Zelllinie mit der Hinterlegungsnummer ATCC HB9679 erhalten werden kann, und einen dritten mAk oder ein Fragment davon, welcher bzw. welches das Epitop erkennt, das durch den mAk LDQ10 erkannt wird, welcher von der Zelllinie mit der Hinterlegungsnummer ATCC HB12417 erhalten werden kann, für eine ausreichende Zeitspanne, um dekorierte Zellen zu erhalten;
d) Ausführen einer immunzytologischen Analyse an den dekorierten Zellen;
e) Bestimmen des Nichtvorhandenseins oder Vorliegens von Blasenkrebs;
wobei der erste und der dritte mAk oder die Fragmente davon mit einem ersten Fluorochrom markiert sind und der zweite mAk oder das Fragment davon mit einem unterschiedlichen Fluorochrom markiert ist und wobei angenommen, dass die Detektion von wenigstens einer positiven Zelle, die mit dem ersten oder dritten mAk oder Fragment davon reagiert, oder die Detektion von wenigstens einer Zelle, die mit dem zweiten mAk oder Fragment davon reagiert, auf Blasenkrebs schließen lässt.

2. Nicht-invasiver Untersuchungstest nach Anspruch 1, wobei die immunologische Zusammensetzung mAk M344, welcher von der Zelllinie mit der Hinterlegungsnummer ATCC HB9678 erhalten werden kann, mAk 19A211, welcher von der Zelllinie mit der Hinterlegungsnummer ATCC HB9679 erhalten werden kann, und mAk LDQ10, welcher von der Zelllinie mit der Hinterlegungsnummer ATCC HB12417 erhalten werden kann, umfasst.

3. Test nach Anspruch 1 oder 2, welcher ferner umfasst, an den fixierten Zellen oder den dekorierten Zellen eine klassische zytologische Analyse auszuführen.

4. Nicht-invasiver Untersuchungstest nach einem der Ansprüche 1 bis 3, wobei angenommen wird, dass die Detektion von wenigstens einer Zelle, die mit einem mAk reagiert, der ein durch die mAk M344 oder LDQ10 erkanntes Epitop erkennt, und die Detektion von wenigstens einer Zelle, die mit einem mAk 19A211 reagiert, auf Blasenkrebs schließen lassen.

5. Nicht-invasiver Untersuchungstest nach einem der Ansprüche 1 bis 3, wobei angenommen wird, dass die Detektion von wenigstens einer Zelle, die mit einem mAk reagiert, der ein durch die mAk M344 oder LDQ10 erkanntes Epitop erkennt, oder die Detektion von wenigstens fünf Zellen, die mit einem mAk 19A211 reagieren, auf Blasenkrebs schließen lässt.

6. Nicht-invasiver Untersuchungstest nach einem der Ansprüche 1 bis 3, wobei angenommen wird, dass die Detektion von wenigstens einer Zelle, die mit einem mAk reagiert, der ein durch mAk M344 oder LDQ10 erkanntes Epitop erkennt, oder die Detektion von wenigstens zehn Zellen, die mit einem mAk 19A211 reagieren, auf Blasenkrebs schließen lässt.

7. Nicht-invasiver Untersuchungsstest nach einem der Ansprüche 1 bis 3, wobei angenommen wird, dass die Detektion von wenigstens fünf Zellen, die mit einem mAk reagieren, der ein durch die mAk M344, LDQ10 oder 19A211 erkanntes Epitop erkennt, auf Blasenkrebs schließen lässt.

8. Nicht-invasiver Untersuchungstest nach einem der Ansprüche 1 bis 3, wobei angenommen wird, dass die Detektion von wenigstens zehn Zellen, die mit einem mAk reagieren, der ein durch die mAks M344, LDQ10 oder 19A211 erkanntes Epitop erkennt, auf Blasenkrebs schließen lässt.

9. Nicht-invasiver Untersuchungstest nach einem der Ansprüche 1-3, wobei die Empfindlichkeit des Tests höher als 80% ist.

10. Nicht-invasiver Untersuchungstest nach einem der Ansprüche 1-3, wobei die Empfindlichkeit des Tests höher als 90% ist.

11. Nicht-invasiver Untersuchungstest nach einem der Ansprüche 1-3, wobei die Empfindlichkeit des Tests höher als 95% ist.

12. Nicht-invasiver Untersuchungstest nach einem der Ansprüche 1-3, wobei die Empfindlichkeit des Tests zwischen 95% und 100% liegt.

13. Test nach einem der Ansprüche 1-12, wobei die biologische Probe eine Urinprobe ist.

14. Verfahren zum Abschätzen des Risikos eines Rezidivs und der Tumorrate bei Blasenkrebs, umfassend:
a) Isolieren von Blasenzellen aus einer biologischen Probe eines Patienten;
b) Fixieren der Zellen, um ihre morphologischen Merkmale zu bewahren;
c) Umsetzen der fixierten Zellen mit einer immunologischen Zusammensetzung, umfassend einen ersten mAk oder ein Fragment davon, welcher bzw. welches das Epitop erkennt, das durch den mAk M344 erkannt wird, welcher von der Zelllinie mit der Hinterlegungsnummer ATCC HB9678 erhalten werden kann, einen zweiten mAk oder ein Fragment davon, welcher bzw. welches das Epitop erkennt, das durch den mAk 19A211 erkannt wird, welcher von der Zelllinie mit der Hinterlegungsnummer ATCC HB9679 erhalten werden kann, und einen dritten mAk oder ein Fragment davon, welcher bzw. welches das Epitop erkennt, das durch den mAk LDQ10 erkannt wird, welcher von der Zelllinie mit der Aufnahmenummer ATCC HB12417 erhalten werden kann; und
d) Abschätzen des Risikos eines Rezidivs und der Tumorrate basierend auf der Reaktivität der mAk mit dem Tumor,
wobei der erste und der dritte mAk oder die Fragmente davon mit einem ersten Fluorochrom markiert sind und der zweite mAk oder das Fragment davon mit einem unterschiedlichen Fluorochrom markiert ist und wobei das Risiko eines Rezidivs des Tumors bestimmt werden kann, indem das Ausmaß an Reaktivität des ersten mAk oder Fragments davon oder des dritten mAk oder Fragments davon, welche mit einer Zunahme der Tumorrate assoziiert ist, mit einer Reaktivität des zweiten mAk mit dem Tumor verglichen wird, wobei diese mit einer Zunahme einer Rezidivgefahr und der Rezidivrate assoziiert ist.

15. Verfahren nach Anspruch 14, wobei die immunologische Zusammensetzung mAk M344, welcher von der Zelllinie mit der Hinterlegungsnummer ATCC HB9678 erhalten werden kann, mAk 19A211, welcher von der Zelllinie mit der Hinterlegungsnummer ATCC HB9679 erhalten werden kann, und mAk LDQ10, welcher von der Zelllinie mit der Hinterlegungsnummer ATCC HB12417 erhalten werden kann, umfasst.

16. Verfahren nach Anspruch 14 oder 15, wobei angenommen wird, dass die Detektion von wenigstens einer positiven Zelle, die mit dem ersten oder dritten mAk reagiert, oder die Detektion von wenigstens einer Zelle, die mit dem zweiten mAk reagiert, auf Blasenkrebs schließen lässt.

17. Verfahren nach Anspruch 14, 15 oder 16, welches ferner umfasst, an den fixierten oder dekorierten Zellen eine klassische zytologische Analyse auszuführen.

18. Verfahren nach einem der Ansprüche 14-17, wobei angenommen wird, dass die Detektion von wenigstens einer Zelle, die mit einem mAk reagiert, der ein durch die mAk M344 oder LDQ10 erkanntes Epitop erkennt, und die Detektion von wenigstens einer Zelle, die mit einem mAk 19A211 reagiert, auf Blasenkrebs schließen lassen.

19. Verfahren nach einem der Ansprüche 14-17, wobei angenommen wird, dass die Detektion von wenigstens einer Zelle, die mit einem mAk reagiert, der ein durch die mAk M344 oder LDQ10 erkanntes Epitop erkennt, oder die Detektion von wenigstens fünf Zellen, die mit einem mAk 19A211 reagieren, auf Blasenkrebs schließen lässt.

20. Verfahren nach einem der Ansprüche 14-17, wobei angenommen wird, dass die Detektion von wenigstens einer Zelle, die mit einem mAk reagiert, der ein durch mAk M344 oder LDQ10 erkanntes Epitop erkennt, oder die Detektion von wenigstens zehn Zellen, die mit einem mAk 19A211 reagieren, auf Blasenkrebs schließen lässt.

21. Verfahren nach einem der Ansprüche 14-17, wobei angenommen wird, dass die Detektion von wenigstens fünf Zellen, die mit einem mAk reagieren, der ein durch die mAk M344, LDQ10 oder 19A211 erkanntes Epitop erkennt, auf Blasenkrebs schließen lässt.

22. Verfahren nach einem der Ansprüche 14-17, wobei angenommen wird, dass die Detektion von wenigstens zehn Zellen, die mit einem mAk reagieren, der ein durch die mAk M344, LDQ10 oder 19A211 erkanntes Epitop erkennt, auf Blasenkrebs schließen lässt.

23. Verfahren nach einem der Ansprüche 14-17, wobei die Empfindlichkeit des Tests höher als 80% ist.

24. Verfahren nach einem der Ansprüche 14-17, wobei die Empfindlichkeit des Tests höher als 90% ist.

25. Verfahren nach einem der Ansprüche 14-17, wobei die Empfindlichkeit des Tests höher als 95% ist.

26. Verfahren nach einem der Ansprüche 14-17, wobei die Empfindlichkeit des Tests zwischen 95% und 100% liegt.

27. Verfahren nach einem der Ansprüche 14-26, wobei die biologische Probe eine Urinprobe ist.

28. Immunologische Zusammensetzung für den Nachweis von Blasenkrebs, umfassend monoklonale Antikörper (mAk) oder ein Fragment davon, welche mit Blasenkrebs assoziierte Antigene erkennen, wobei die mAk oder das Fragment davon einen mAk oder ein Fragment davon umfassen, welcher bzw. welches das Epitop erkennt, das durch den mAk M344 erkannt wird, welcher von der Zelllinie mit der Hinterlegungsnummer ATCC HB9678 erhalten werden kann, den mAk 19A211, welcher von der Zelllinie mit der Hinterlegungsnummer ATCC HB9679 erhalten werden kann, und den mAk LDQ10, welcher von der Zelllinie mit der Hinterlegungsnummer ATCC HB12417 erhalten werden kann, wobei der mAk oder das Fragment davon, welcher bzw. welches das durch den mAk M344 erkannte Epitop erkennt, und der mAk oder das Fragment davon, welcher bzw. welches das durch den mAk LDQ10 erkannte Epitop erkennt, mit einem ersten Fluorochrom markiert sind und der mAk oder das Fragment davon, welcher bzw. welches das durch den mAk 19A211 erkannte Epitop erkennen, mit einem unterschiedlichen Fluorochrom markiert ist.

29. Immunologische Zusammensetzung nach Anspruch 28, umfassend mAk M344, welcher von der Zelllinie mit der Hinterlegungsnummer ATCC HB9678 erhalten werden kann, mAk 19A211, welcher von der Zelllinie mit der Hinterlegungsnummer ATCC HB9679 erhalten werden kann, und mAk LDQ10, welcher von der Zelllinie mit der Hinterlegungsnummer ATCC HB12417 erhalten werden kann.

30. Diagnostischer Kit, welcher die Zusammensetzung von Anspruch 28 oder 29 umfasst.

## Revendications

1. Test d'investigation non invasif pour la détection d'un cancer de la vessie comprenant les étapes consistant à ;
a) collecter des cellules de la vessie dans un échantillon biologique ;
b) fixer lesdites cellules :
c) mettre en réaction lesdites cellules fixées avec une composition immunologique comprenant un premier AcM ou fragment de celui-ci reconnaissant les épitopes reconnus par l'AcM M344 pouvant être obtenu à partir de la lignée cellulaire ayant le numéro d'accès ATCC HB9678, un deuxième AcM ou fragment de celui-ci reconnaissant l'épitope reconnu par l'AcM 19A211 pouvant être obtenu à partir de la lignée cellulaire ayant le numéro d'accès ATCC HB9679, et un troisième AcM ou fragment de celui-ci reconnaissant l'épitope reconnu par l'AcM LDQ10 pouvant être obtenu à partir de la lignée cellulaire ayant le numéro d'accès ATCC HB12417 pendant une durée suffisante pour obtenir des cellules marquées ;
d) réaliser une analyse immunocytologique sur lesdites cellules marquées ; et
e) évaluer l'absence ou la présence du cancer de la vessie,
dans lequel lesdits premier et troisième AcM ou fragment de ceux-ci sont marqués par un premier fluorochrome et ledit deuxième AcM ou fragment de celui-ci est marqué avec un fluorochrome différent et dans lequel la détection d'au moins une cellule positive réagissant avec ledit premier ou troisième AcM ou fragment de ceux-ci ou la détection d'au moins une cellule réagissant avec ledit deuxième AcM ou fragment de celui-ci sont considérées comme indiquant un cancer de la vessie.

2. Test d'investigation non invasif selon la revendication 1, dans lequel ladite composition immunologique comprend l'AcM M344 pouvant être obtenu à partir de la lignée cellulaire ayant le numéro d'accès ATCC HB9678, l'AcM 19A211 pouvant être obtenu à partir de la lignée cellulaire ayant le numéro d'accès ATCC HB9679 et l'AcM LDQ10 pouvant être obtenu à partir de la lignée cellulaire ayant le numéro d'accès ATCC HB12417.

3. Test selon les revendications 1 ou 2, comprenant en outre l'étape consistant à réaliser une analyse cytologique classique sur lesdites cellules fixées ou lesdites cellules marquées.

4. Test d'investigation non invasif selon l'une quelconque des revendications 1-3, dans lequel la détection d'au moins une cellule réagissant avec un AcM reconnaissant un épitope reconnu par les AcM M344 ou LDQ10 et la détection d'au moins une cellule réagissant avec un AcM 19A211 sont considérées comme indiquant un cancer de la vessie.

5. Test d'investigation non invasif selon l'une quelconque des revendications 1-3, dans lequel la détection d'au moins une cellule réagissant avec un AcM reconnaissant un épitope reconnu par les AcM M344 ou LDQ10 ou la détection d'au moins 5 cellules réagissant avec un AcM 19A211 sont considérées comme indiquant un cancer de la vessie.

6. Test d'investigation non invasif selon l'une quelconque des revendications 1-3, dans lequel la détection d'au moins une cellule réagissant avec un AcM reconnaissant un épitope reconnu par les AcM M344 ou LDQ10 ou la détection d'au moins 10 cellules réagissant avec un AcM 19A211 sont considérées comme indiquant un cancer de la vessie.

7. Test d'investigation non invasif selon l'une quelconque des revendications 1-3, dans lequel la détection d'au moins 5 cellules réagissant avec un AcM reconnaissant un épitope reconnu par les AcM M344, LDQ10 ou 19A211 est considérée comme indiquant un cancer de la vessie.

8. Test d'investigation non invasif selon l'une quelconque des revendications 1-3, dans lequel la détection d'au moins 10 cellules réagissant avec un AcM reconnaissant un épitope reconnu par les AcM M344, LDQ10 ou 19A211 est considérée comme indiquant un cancer de la vessie.

9. Test d'investigation non invasif selon l'une quelconque des revendications 1-3, dans lequel la sensibilité dudit test est supérieure à 80 %.

10. Test d'investigation non invasif selon l'une quelconque des revendications 1-3, dans lequel la sensibilité dudit test est supérieure à 90 %

11. Test d'investigation non invasif selon l'une quelconque des revendications 1-3, dans lequel la sensibilité dudit test est supérieure à 95 %.

12. Test d'investigation non invasif selon l'une quelconque des revendications 1-3, dans lequel la sensibilité dudit test est entre 95 % et 100 %.

13. Test selon l'une quelconque des revendications 1-12, dans lequel ledit échantillon biologique est un échantillon d'urine.

14. Procédé pour estimer le risque de récidive et le taux de tumeur dans le cancer de la vessie comprenant les étapes consistant à :
a) isoler des cellules de vessie dans un échantillon biologique d'un patient ;
b) fixer lesdites cellules de manière à préserver leurs caractéristiques morphologiques ;
c) mettre en réaction lesdites cellules fixées avec une composition immunologique comprenant un premier AcM ou fragment de celui-ci reconnaissant l'épitope reconnu par l'AcM M344 pouvant être obtenu à partir de la lignée cellulaire ayant le numéro d'accès ATCC HB9678, un deuxième AcM ou fragment de celui-ci reconnaissant l'épitope reconnu par l'AcM 19A211 pouvant être obtenu à partir de la lignée cellulaire ayant le numéro d'accès ATCC HB9679, et un troisième AcM ou fragment de celui-ci reconnaissant l'épitopc reconnu par l'AcM LDQ10 pouvant être obtenu à partir de la lignée cellulaire ayant le numéro d'accès ATCC HB12417 ; et
d) estimer le risque de récidive et le taux de tumeur, fondés sur la réactivité desdits AcM avec ladite tumeur,
dans lequel lesdits premier et troisième AcM ou fragment de ceux-ci sont marqués avec un premier fluorochrome et ledit deuxième AcM ou fragment de celui-ci est marqué avec un fluorochrome différent et dans lequel ledit risque de récidive de la tumeur peut être déterminé en comparant la quantité de réactivité dudit premier AcM ou fragment de celui-ci, ou dudit troisième AcM ou fragment de celui-ci qui est associée à une augmentation du taux de tumeur, la réactivité dudit deuxième AcM avec la tumeur étant associée à une augmentation du risque de récidive et du taux de récidive.

15. Procédé selon la revendication 14, dans lequel ladite composition immunologique comprend l'AcM M344 pouvant être obtenu à partir de la lignée cellulaire ayant le numéro d'accès ATCC HB9678, l'AcM 19A211 pouvant être obtenu à partir de la lignée cellulaire ayant le numéro d'accès ATCC HB9679 et l'AcM LDQ10 pouvant être obtenu à partir de la lignée cellulaire ayant le numéro d'accès ATCC HB12417.

16. Procédé selon la revendication 14 ou 15, dans lequel la détection d'au moins une cellule positive réagissant avec ledit premier ou troisième AcM ou la détection d'au moins une cellule réagissant avec ledit deuxième AcM sont considérées comme indiquant un cancer de la vessie.

17. Procédé selon la revendication 14, 15 ou 16, comprenant en outre la réalisation d'une analyse cytologique classique sur lesdites cellules fixées ou marquées.

18. Procédé selon l'une quelconque des revendications 14-17, dans lequel la détection d'au moins une cellule réagissant avec un AcM reconnaissant un épitope reconnu par les AcM M344 ou LDQ10 ou la détection d'au moins une cellule réagissant avec un AcM 19A211 sont considérées comme indiquant un cancer de la vessie.

19. Procédé selon l'une quelconque des revendications 14-17, dans lequel la détection d'au moins une cellule réagissant avec un AcM reconnaissant un épitope reconnu par les AcM M344 ou LDQ10 ou la détection d'au moins 5 cellules réagissant avec un AcM 19A211 sont considérées comme indiquant un cancer de la vessie.

20. Procédé selon l'une quelconque des revendications 14-17, dans lequel la détection d'au moins une cellule réagissant avec un AcM reconnaissant un épitope reconnu par les AcM M344 ou LDQ10 ou la détection d'au moins 10 cellules réagissant avec un AcM 19A211 sont considérées comme indiquant un cancer de la vessie.

21. Procédé selon l'une quelconque des revendications 14-17, dans lequel la détection d'au moins 5 cellules réagissant avec un AcM reconnaissant un épitope reconnu par les AcM M344, LDQ10 ou 19A211 est considérée comme indiquant un cancer de la vessie.

22. Procédé selon l'une quelconque des revendications 14-17, dans lequel la détection d'au moins 10 cellules réagissant avec un AcM reconnaissant un épitope reconnu par les AcM M344, LDQ10 ou 19A211 est considérée comme indiquant un cancer de la vessie.

23. Procédé selon l'une quelconque des revendications 14-17, dans lequel la sensibilité dudit test est supérieure à 80 %.

24. Procédé selon l'une quelconque des revendications 14-17, dans lequel la sensibilité dudit test est supérieure à 90 %.

25. Procédé selon l'une quelconque des revendications 14-17, dans lequel la sensibilité dudit test est supérieure à 95 %.

26. Procédé selon l'une quelconque des revendications 14-17, dans lequel la sensibilité dudit test est comprise entre 95 % et 100 %.

27. Procédé selon l'une quelconque des revendications 14-26, dans lequel l'échantillon biologique est un échantillon d'urine.

28. Composition immunologique pour la détection du cancer de la vessie, comprenant des anticorps monoclonaux (AcM) ou un fragment de ceux-ci reconnaissant des antigènes associés au cancer de la vessie, dans laquelle lesdits AcM ou ledit fragment de ceux-ci comprennent un AcM ou un fragment de celui-ci reconnaissant l'épitope reconnu par l'AcM M344 pouvant être obtenu à partir de la lignée cellulaire ayant le numéro d'accès ATCC HB9678, l'AcM 19A211 pouvant être obtenu à partir de la lignée cellulaire ayant le numéro d'accès ATCC HB9679, et l'AcM LDQ10 pouvant être obtenu à partir de la lignée cellulaire ayant le numéro d'accès ATCC HB12417, dans laquelle ledit AcM ou fragment de celui-ci reconnaissant l'épitope reconnu par l'AcM M344 et ledit AcM ou fragment de celui-ci reconnaissant l'épitope reconnu par l'AcM LDQ10 sont marqués avec un premier fluorochrome et ledit AcM ou fragment de celui-ci reconnaissant l'épitope reconnu par l'AcM 19A211 est marqué avec un fluorochrome différent.

29. Composition immunologique de la revendication 28 comprenant l'AcM M344 pouvant être obtenu à partir de la lignée cellulaire ayant le numéro d'accès ATCC HB9678, l'AcM 19A211 pouvant être obtenu à partir de la lignée cellulaire ayant le numéro d'accès ATCC HB9679, et l'AcM LDQ10 pouvant être obtenu à partir de la lignée cellulaire ayant le numéro d'accès ATCC HB12417.

30. Kit de diagnostic comprenant la composition de la revendication 28 ou 29.
